# EUROPEAN PATENT SPECIFICATION

(11) **EP 2 861 285 B1**
(45) Date of publication and mention of the grant of the patent: **25.11.2020**
(21) Application number: 13803881.5
(22) Date of filing: 13.06.2013
(51) Int. Cl.: A61M 15/00

(54) **METERED DOSE DISPENSING VALVE**
SPENDERVENTIL FÜR ABGEMESSENE DOSIERUNG
CLAPET DE DISTRIBUTION DOSEUR

(30) Priority: 14.06.2012 GB 201210580
(43) Date of publication of application: 22.04.2015
(73) Proprietor: 3M Innovative Properties Company, St. Paul, MN 55133-3427 (US)
(72) Inventor: STUART, Adam J., Bracknell Berkshire RG12 8HT (GB); HODSON, Peter D., Bracknell Berkshire RG12 8HT (GB); HOWGILL, Stephen J., Thurcaston Leicestershire LE7 7JL (GB); OAKLEY, Matthew A., Market Harborough Leicestershire LE16 9AS (GB); GREENLEAF, David J., Bracknell Berkshire RG12 8HT (GB)
(74) Representative: Vossius & Partner Patentanwälte Rechtsanwälte mbB
(86) International application number: PCT/US2013/045549
(87) International publication number: WO 2013/188609

(56) References cited:
- EP-A2- 0 224 380
- US-A- 5 772 085
- US-A1- 2004 139 966
- US-A1- 2008 067 199
- US-A1- 2010 300 437
- US-A1- 2010 300 437

## Description

### Cross Reference to Related Applications

This application claims priority to United Kingdom Application No. 1210580.5, filed June 14, 2012.

### Field

The present invention relates to metered dose dispensing valves and in particular to metered dose dispensing valves suitable for use with metered dose inhalers.

### Background

Asthma and other respiratory diseases have long been treated by the inhalation of appropriate medicament. Pulmonary inhalation is also becoming an attractive route of administration of medicaments that may be difficult to deliver orally such as proteins and peptides.

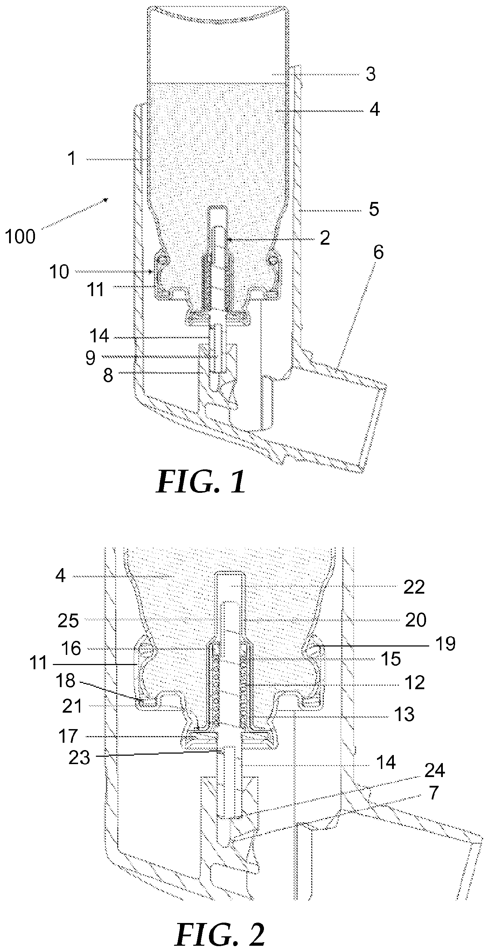

A widely used and convenient choice of pulmonary drug delivery has been the inhalation of medicament from an aerosol created by a pressurized metered dose inhaler (pMDI). As shown in Figure 1, a pMDI (100) typically comprises a canister (10) including a metered dose dispensing valve (2) mounted via a ferrule (11) onto an aerosol container or vial (1) defining in part a formulation chamber (3) filled with medicinal inhalation formulation (4) and an actuator (5) including a mouthpiece (6). (In an alternative form, suitable for nasal drug delivery, the actuator may comprise a nosepiece rather than a mouthpiece.) The canister is contained within the actuator by inserting the valve stem (14) of the valve, which protrudes outside the ferrule, into a support block (8) of the actuator. The valve stem has a dispensing passage (9) which allows for passage of substance from a metering chamber of the valve out through the valve stem and actuator nosepiece or mouthpiece to the user. Medicinal aerosol formulations typically comprise medicament either in solution or as particles suspended in liquefied propellant(s), e.g. CFC propellant(s) and more recently non-CFC propellant(s), such as 1,1,1,2-tetrafluoroethane (HFA134a) and/or 1,1,1,2,3,3,3-heptafluoropropane (HFA227). If desired and/or deemed necessary, the formulation may comprise other components, such as excipients, co-solvents, and suspending aids. Depending on the particular metered dose valve and/or filling system, medicament formulation may be filled into the pMDI either by cold-filling (in which chilled formulation is filled into the vial and subsequently the metered dose valve is fitted onto the vial) or by pressure filling (in which the metered dose valve is fitted onto the vial and then formulation is pressure filled through the valve into the vial).

Typical commercial pMDI metering valves comprise seven or eight or even more components. An example is the 3M Spraymiser™ valve by 3M Company, St Paul, Minnesota, USA (Figures 1 and 2). Referring to Figure 2, this valve in its usual form comprises eight (or optionally nine) components: a first valve body (13) defining in part a metering chamber (12), a second valve body (20) defining in part a pre-metering region (22) and acting in this valve as a bottle emptier, a valve stem (14), a biasing member in the form of a coil spring (15), an inner seal (16), an outer seal (17), a ferrule (11) and a gasket seal (18) (and an optional O-ring (19)). Although most of the individual components can be made cheaply, the number of them, together with a need for complicated manipulation during their assembly and a need for quite accurate alignment in their assembly, means that overall ex-factory costs can be relatively high, at least in terms of the prices that the rapidly developing markets of India, China and other eastern countries are willing to pay. This relatively high cost of valves may act as a barrier to their acceptance in highly price-sensitive markets. Accordingly marketing of associated pMDIs is limited, meaning many asthma sufferers in poorer countries are denied the common, accepted treatments that asthma sufferers would expect to receive in more affluent parts of the world.

Besides cost issues, it has been observed that issues related to component alignment and guidance and complex dimensional tolerance stack-up factors may generate secondary potential issues with valve and pMDI performance, such as poor pressure-filling performance, high firing forces and/or inadequacies in stem return forces, as well as undesirable propellant leakage and moisture penetration rates.

The following documents disclose types of valve, some of which might be intended to be low cost and/or to have a limited number of components: GB 1054307 (Riker), GB 2361228 (Groeger), US 3019947 (Gorman), EP 816 255 (Hildebrandt), GB 1115926 (Nadal), CH 428604 (Trautmann), GB 2300674 (Lacout), US 3521859 (Gronemeyer), US 3642180 (Lehmann), US 3862741 (Steiman & Beres), US 3982674 (Mildern), US 4471893 (Knickerbocker), US 4477001 (Galia), US 4852807 (Stoody), US 4887743 (Blake), US 5775545 (Sullivan), WO 95/28620 (Keller), and WO 02/02435 (Corba). Note that not all these valves are metering valves.

US 2010/0300437 A1 discloses the use of metal powder injection molding for the manufacture of a metal valve component of a metered dose dispensing valve for use in a medicinal pressurized metered dose dispenser.

EP 0 224 380 A2 discloses valve assemblies suitable for use with a beer keg.

### Summary of Invention

A metered dose dispensing valve as recited in claim 1 is provided. The dependent claims define embodiments.

Although a number of documents seemingly suggest low cost valves and/or valves with few components, presently there is no such valve with notable success on the market. Without wanting to be bound to a particular theory, it seems that the lack of success may be related to the fact that previously suggested valves show significant dissimilarities to industry-standard valves in the way that they interface with aerosol containers or actuator support blocks and/or perhaps in fact exhibit a higher level of complexity and/or lower levels of physical or operational robustness and/or provide poor valve performance (e.g. very variable dose volumes, high stem friction, high leakage rates, etc).

Accordingly there is an ongoing need and a demand for inexpensive pMDI valves of good quality and high reliability. It would be advantageous to overcome or mitigate issues in metering valves that can be made for very low cost, that can be pressure-filled, and that can interface in a conventional way to both the pMDI actuator and the typical aerosol container used in a pMDI (e.g. using a hollow male valve stem and a mechanically crimpable ferrule). In addition, it would be desirable to provide such a valve that would operate in the same way that conventional pMDI valves do: i.e. dispensing a metered dose of formulation along the valve stem bore when the stem is pushed inwardly (i.e. towards the aerosol container) along its longitudinal axis, and then resetting (with the stem moving back outwardly to its starting point) when the stem is released by the patient.

In one aspect of the present invention there is provided a metered dose dispensing valve for dispensing metered volumes of an aerosol formulation from an aerosol container, said valve comprising
a first valve body defining in part a metering chamber;
a valve stem passing axially through the metering chamber, movable relative to the chamber between non-dispensing and dispensing positions, and biased from its dispensing position towards its non-dispensing position by a compliant biasing member; and
a second valve body defining at least in part a pre-metering region,
wherein the biasing member has at least one portion engaging the valve stem, thereby imparting a bias thereto, and at least one other portion being anchored to the second valve body; wherein, in use, the valve stem is moved axially against said bias from its non-dispensing position into its dispensing position and upon release the valve stem moves under the action of the bias from its dispensing position back to its non-dispensing position; and wherein said biasing member and second valve body are integrally provided in a single component.

Surprisingly we have found that by combining the compliant biasing member and the second valve body into an integral single component it is possible to provide a desirable metered dose dispensing valve for use in inhalation devices or for use in the provision of canisters (i.e. an aerosol container, filled or not yet filled with aerosol formulation, plus a valve) targeted for use in inhalation devices. Valves described herein are particularly desirable for dispensing metered volumes of pressurized aerosol formulations, and thus particularly suitable for use in pressurized metered dose inhalers or for use in the provision of canisters targeted for use in pressurized metered dose inhalers. In particular, the provision of such an integral single component allows one to avoid or minimize manufacturing costs and/or performance issues and/or dimensional tolerance issues associated with the assembly and alignment of a coil spring into a valve. In addition, valves described herein have a valve stem with axial travel distances to and from its dispensing and non-dispensing positions, wherein in its dispensing position a metered dose may be delivered and in its non-dispensing position the metering chamber may be refilled via the pre-metering region, said axial travel distances being very similar to those of industry-standard pMDI valves. Accordingly such valves are advantageously broadly similar to those of industry-standard valves, helping to make them readily compatible with existing actuators, aerosol containers and dose indicators, and making them acceptable to the industry as a stand-alone component offering.

Favorably, the valve operates similarly to industry-standard push-to-fire pMDI valves. Moreover, favorably the valve is configured and arranged such that, in use, when the valve stem is in its non-dispensing position, the pre-metering region is in communication either continuously or transiently with the contents of the aerosol container and the metering chamber is in communication at least transiently with the pre-metering region to allow substance to pass from the aerosol container to the metering chamber, and when the valve stem is in its dispensing position, the pre-metering region is isolated from the metering chamber and a communication path is provided between the metering chamber and the outside of the valve and aerosol container assembly, said path being either continuously or transiently open to allow substance to pass from the metering chamber to the outside of the valve and aerosol container assembly. The skilled person will understand that the terms 'dispensing position' and 'non-dispensing position' actually each refer to a range of spatial positions of the valve stem over its span of axial movement. The term 'rest position' refers to one specific non-dispensing position. The terms 'continuously open' and 'transiently open' in the context of the communication path may refer to both the case where the communication path is open continuously or transiently spatially or is open continuously or transiently temporally during valve stem operation.

Favorably the valve is designed to be similar to industry-standard push-to-actuate pMDI valves in that, in use, the valve stem is moved axially, e.g. by the user or a breath actuated mechanism, inwardly towards the aerosol container from its non-dispensing position to its dispensing position and upon release moves outwardly under the action of the bias from its dispensing position.

Favorably the second valve body, which is desirably formed with a cage or cage-like structure, acts among other things as a fixed support during valve operation for the biasing member that in turn acts to apply a bias to the valve stem. The compliant biasing member may favorably be a spring member. The term "spring" is generally understood to be an elastic object (e.g. member) used to store mechanical energy, whereby when the object is compressed or stretched, the force it exerts is proportional (or approximately proportional) to its change in length. Alternatively, a torsion spring could be used.

To further facilitate minimization of the overall number of components and/or any or all performance issues outlined above, desirably the valve stem is integrally provided together with the biasing member and second valve body in said single component. In other words, in such embodiments, the second valve body, biasing member and valve stem are integrally provided in a single component.

Integral single components including the second valve body, biasing member and, in more favorable embodiments, the valve stem, may favorably comprise a polymeric material, for example with one or more polymeric materials in a composite and/or including other materials such as fillers and/or reinforcing agents in one or more of the polymeric materials. Such components may be favorably molded, in particular via one-shot molding or two or more shot moldings. Alternatively such integral single component may favorably comprise metal and a polymeric material, for example when the compliant biasing member is made of or includes a metal sub-structure. Herein the single component may be desirably molded by insert molding in conjunction with one-shot or two or more shot polymeric molding (e.g. insert a metal spring into the appropriate portion of the component mold and then mold and as applicable over-mold the polymeric elements of the component). The use of metal per se or a substructure of metal (or other suitable materials) may facilitate avoidance or reduction of any tendency of the compliant biasing member to undergo stress relaxation or creep.

Favorably the valve further comprises an inner seal and an outer seal. The inner seal is generally located relative to the canister towards the interior, while the outer seal is generally located further away from the interior. The outer seal is favorably located at or near the open end of the first valve body away from the interior. The inner seal is favorably located at or near the open end of the first valve body towards the interior.

Favorably the valve stem comprises a dispensing passage, wherein the valve stem is movable relative to the inner and outer seals, such that,
in the non-dispensing position of the valve stem, the dispensing passage is isolated from the metering chamber, and a communication path is provided between the aerosol container and the metering chamber, said path being either continuously or transiently open to allow substance to pass from the aerosol container to the metering chamber, and
in the dispensing position of the valve stem, said communication path between the aerosol container and the metering chamber is closed and the dispensing passage is in communication, either continuously or transiently, with the metering chamber to allow substance to be dispensed from the metering chamber through the dispensing passage.

Both the inner and outer seals may be provided as separate components, or alternatively to facilitate minimization of manufacturing costs and/or any or all performance issues outlined above, the first valve body and the inner seal or the outer seal or both seals may be advantageously integrally provided in a single component. This second integral component may be composed of a polymeric material, for example with one or more polymeric materials in a composite and/or including other materials such as fillers and/or reinforcing agents in one or more of the polymeric materials. This second component may be molded, in particular molded via one-shot molding or two or more shot molding.

In alternative embodiments including inner and outer seals, the inner seal may provided as an integral member of the integral single component comprising the second valve body and biasing member ("first integral component"), while the first valve body and outer seal are either provided separately or are provided integrally in a single second component.

Valves described herein desirably further comprise a ferrule for securely attaching the valve onto the aerosol container. The ferrule may be provided as a separate component or alternatively to facilitate minimization of overall number of components, the ferrule may be provided as an integral part of the second integral component.

Valves described herein desirably further comprise a gasket seal. The gasket seal generally facilitates sealing between the valve and the aerosol container when the valve is mounted onto the aerosol container. The gasket seal may be provided as a separate component, e.g. in the form of a rubber or elastomeric ring, or alternatively to facilitate minimization of overall number of components, the gasket seal may be provided as an integral part of the first integral component or as an integral part of the second integral component or as an integral part of the ferrule. Typically, the gasket seal is in the form of an annular ring with a generally rectangular cross-sectional profile.

Another aspect of the present invention is the provision of a canister comprising an aerosol container and a valve described herein, in particular a canister filled with an aerosol formulation, more particularly a medicinal aerosol formulation, even more particularly a pressurized, medicinal aerosol formulation, and most particularly a pressurized, medicinal aerosol formulation comprising medicament and a propellant, said propellant comprising HFA 134a and/or HFA 227.

Another aspect of the present invention is the provision of a medicinal delivery device comprising a valve described herein or a canister described herein. Advantageously the device is an inhalation device, in particular a pressurized medicinal inhalation device.

The above summary of the present invention is not intended to describe each disclosed embodiment or every implementation of the present invention. The description that follows more particularly exemplifies illustrative embodiments. Also further embodiments are described in dependent claims. In several places throughout the application, guidance is provided through lists of examples, which examples can be used individually and in various combinations. In each instance, the recited list serves only as a representative group and should not be interpreted as an exclusive list.

### Brief Description of Drawings

The invention will now be described with reference to the accompanying drawings in which:
Figure 1 represents a cross-sectional illustration of a pressurized metered dose inhaler known in the art, while Figure 2 represents an enlarged partial view of the inhaler shown in Figure 1.
Figures 3, 4 and 5 represent illustrations of an exemplary metered dose dispensing valve in accordance to the invention described herein, being a lateral view of a sectioned valve (Figure 3); an isometric view of a sectioned valve (Figure 4), and an isometric view of a sectioned valve and an end of a sectioned aerosol container (Figure 5).
Figures 6, 7 and 8 represent cross-sectional views of a portion of the exemplary valve of Figures 3-5 illustrating positioning in its rest position (Figure 6), its firing position (Figure 8), and a position therebetween (Figure 7).

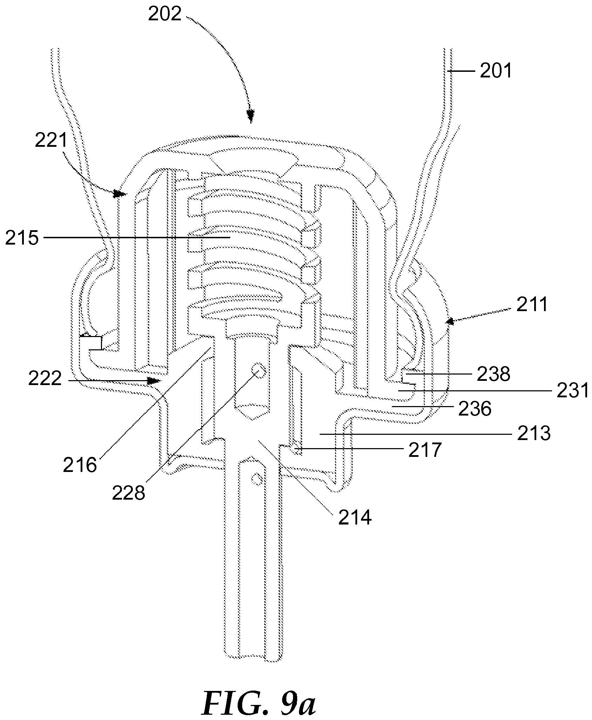

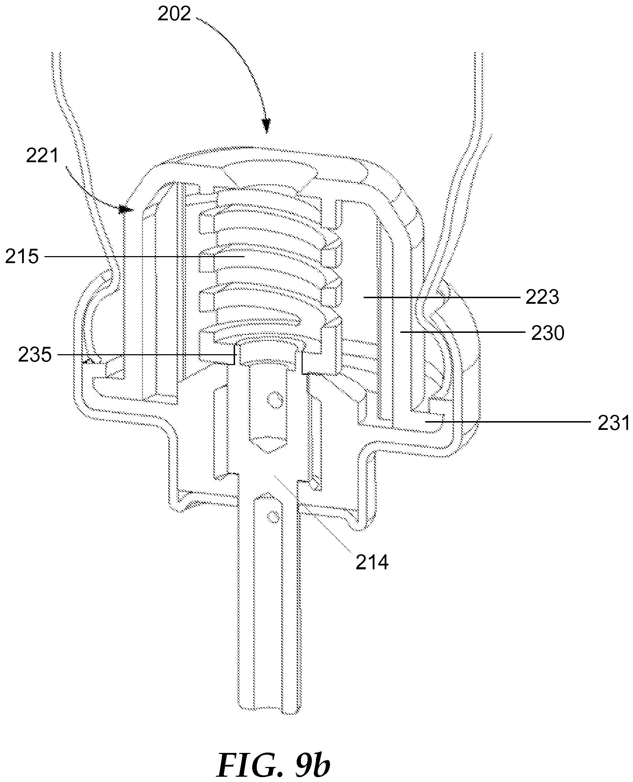

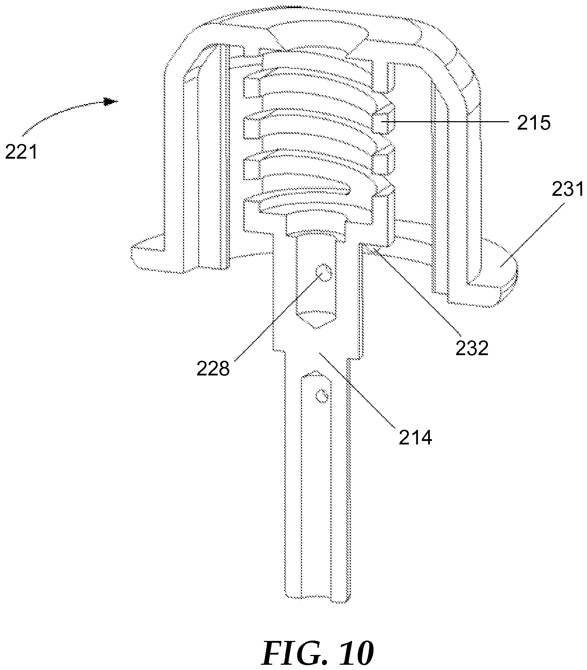

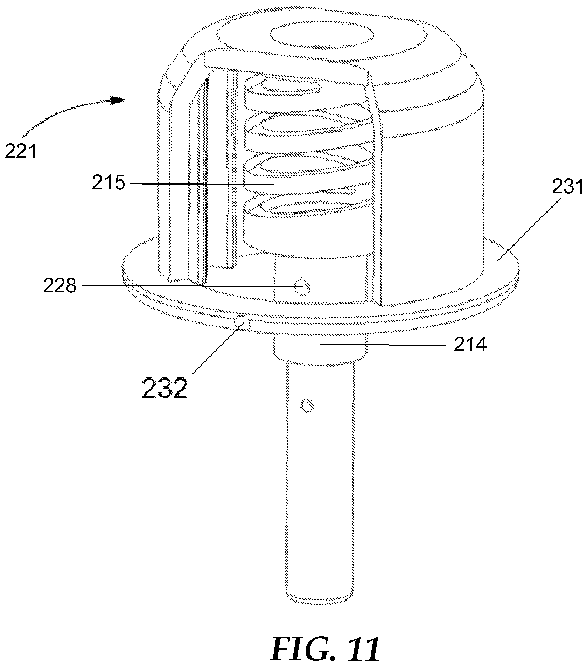

Figures 9a, 10 and 11 represent illustrations of another exemplary metered dose dispensing valve in accordance to the invention described herein, being an isometric view of a sectioned valve and an end of an aerosol container (Figure 9a), an isometric view of a sectioned single integrally provided second valve body, biasing member and valve stem component (Figure 10), and an isometric view of that component (Figure 11). Figure 9b represents an illustration of a different variant of the valve of Figures 9a, 10 and 11, wherein the second valve body and biasing member are provided as a single integral component but the valve stem is provided as a separate component.
Figures 12 to 15 represent schematic illustrations showing views of further exemplary embodiments of single integrally provided second valve body, biasing member and valve stem components in accordance to the invention described herein.
Figure 16 represent an illustration showing yet another exemplary embodiment of a metered dose dispensing valve in accordance to the invention, being a vertical cross section of a valve and an end of an aerosol container.
Figures 17 and 18 represent illustrations of a further exemplary embodiment of a metered dose dispensing valve in accordance to the invention, being isometric views showing sectioned the valve and an end of an aerosol container, the valve being in its non-dispensing and dispensing positions, respectively.
Figures 19 to 21 represent cross-sections of the lower parts of yet three further exemplary embodiments of valves of metered dose valve in accordance with the invention, all operating on a "fast-fill, fast-empty" principle and all shown in their rest positions.
Figure 22 illustrates an alternative exemplary embodiment of the first valve body.

In the description that follows, terms such as 'top', 'bottom', 'above', 'below', etc, refer only to features as shown in the Figures, and no restriction as to orientation of use, etc, is intended. Not all Figures are to the same scale.

### Detailed Description

It is to be understood that the present invention covers all combinations of particular, suitable, desirable, favorable, advantageous and preferred aspects of the invention described herein.

As discussed *supra*, Figure 1 and 2 show an exemplary, well known pressurized metered dose inhaler (Figure 1) or a detailed portion thereof (Figure 2). In particular, Figure 1 shows a metered dose canister (10) including an aerosol container (1) fitted with a metered dose valve (2) (shown in its resting position) as part of a metered dose dispenser (100), in particular an inhaler. The individual parts of the valve have been discussed *supra.* In operation, medicament formulation (4) can pass from the formulation chamber (3) into a pre-metering region (22) provided between the second valve body (20) housing and the first valve body (13) through an annular space (21) between a flange of the second valve body and the first valve body. To actuate (fire) the valve to deliver a dose of medicament formulation, the valve stem (14) is pushed inwardly relative to the aerosol container from its resting position shown in Figures 1 and 2, allowing formulation to pass from the metering chamber (12) through a side hole (23) in the valve stem (14) and through a stem outlet (24) out through an actuator nozzle (7) and then out to the patient. When the valve stem (14) is released, medicament formulation enters into the valve, in particular into the pre-metering chamber (22), through the annular space (21) and thence from the pre-metering chamber (22) through a groove (25) in the valve stem (14) past the inner seal (16) into the metering chamber (12). Because such valves retain the next dose of medication formulation in the metering chamber (12) between actuations, they are sometimes referred to as "retention valves". Retention valves represent the largest sector of the pMDI valve market.

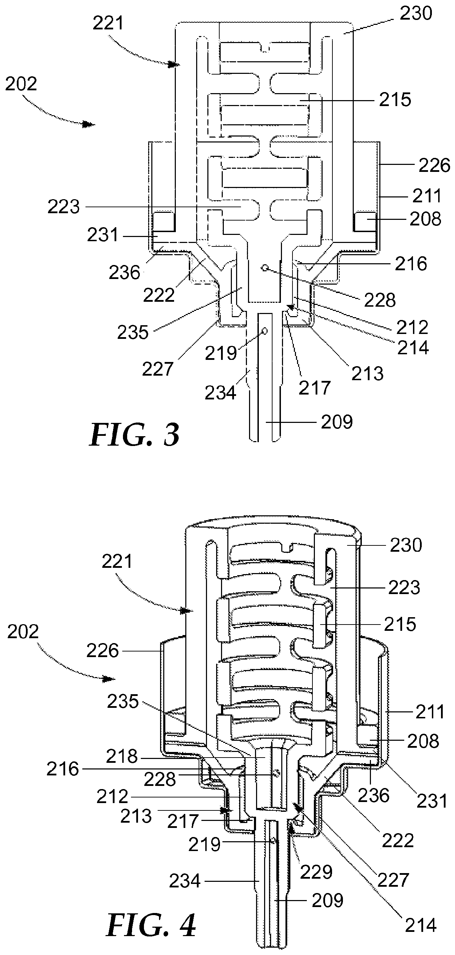

Figures 3 to 21 provide illustrations of a number of exemplary embodiments of metered dose dispensing valves (or if applicable, portions thereof) in accordance with the invention described herein. In these Figures, like reference numerals will denote similar or equivalent, but not necessarily identical, components or features.

Metered dose dispensing valves for dispensing metered volumes of a pressurized aerosol formulation from an aerosol container described herein comprise a first valve body defining in part a metering chamber and a valve stem passing axially through the metering chamber, movable relative to the chamber between non-dispensing and dispensing positions, and biased from its dispensing position towards its non-dispensing position by a compliant biasing member. The volume of the metering chamber (and thus the dose metered upon actuation) may favorably be within the range 25µl to 150µl, and more particularly 50µl to 65µl (end points inclusive for both named ranges). Desirably the first valve body has two open ends through which the valve stem passes, wherein relative to an affixed aerosol container, the first end is located towards the interior of the container and the second end away from the interior of the container. In use, the valve stem is moved axially against said bias from its non-dispensing position into its dispensing position and upon release the valve stem moves outwardly under the action of the bias from its dispensing position back to its non-dispensing position.

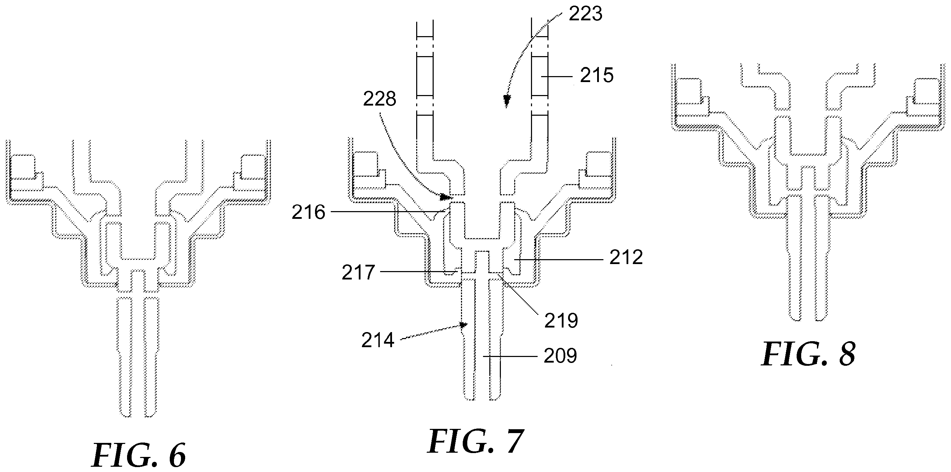

This can be better understood for example in reference to an exemplary embodiment shown in the Figures. Making reference to the view and embodiment shown in Figure 3, it can be seen that the embodiment includes a first valve body (213), defining a metering chamber (212), with inner and outer open ends. It also comprises a valve stem (214) passing axially through the openings in the first valve body. As will be described in more detail below in conjunction with Figures 6 to 8 *infra*, the valve stem (214) is movable relative to the chamber between non-dispensing and dispensing (firing) positions. The valve stem (214) is biased from its dispensing position towards its non-dispensing position by a compliant biasing member (215), desirably in the form of a spring member. As can be appreciated from the illustrations of this exemplary embodiment, the compliant biasing member (215) is provided as a compression spring member.

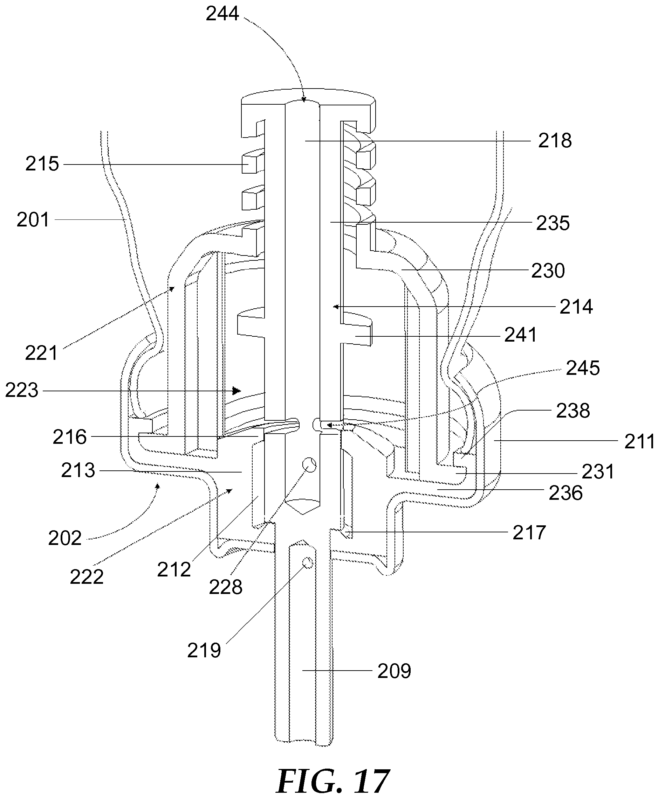

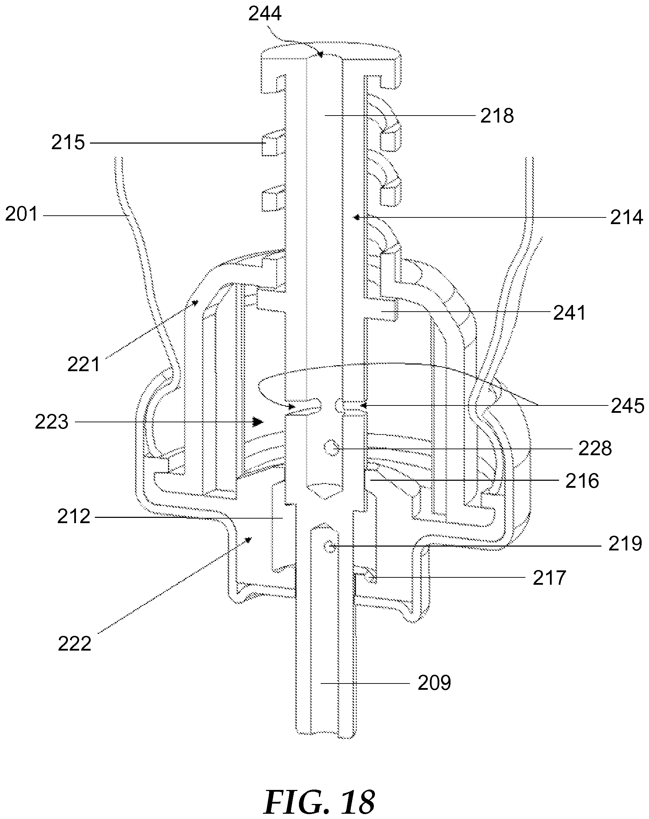

Metered dose dispensing valves for dispensing metered volumes of a pressurized aerosol formulation from an aerosol container described herein further comprise a second valve body defining at least in part a pre-metering region. The second valve body defines a space near the metering chamber to provide a pre-metering region such that the contents of the aerosol container will pass through the pre-metering region to the metering chamber, in particular the contents of the aerosol container will be fed directly and/or indirectly through the pre-metering region to the metering chamber. Advantageously the pre-metering region is located near the inner end of the metering chamber. In one of the exemplary embodiments to be discussed *infra* and shown in Figures 17 and 18, it will be noted that some of the contents of the aerosol container may be fed through a tube which passes through the pre-metering region to reach the metering chamber, i.e. may be fed indirectly through the pre-metering region.

As mentioned above, metered dose dispensing valves for dispensing metered volumes of a pressurized aerosol formulation from an aerosol container described herein comprise a compliant biasing member. The compliant biasing member and second valve body are integrally provided in a single component. As mentioned above, the compliant biasing member imparts a bias onto the valve stem biasing the valve stem towards its non-dispensing position. Typically the second valve body provides fixed support for the compliant biasing member. Accordingly the compliant biasing member has at least one portion engaging the valve stem and at least one other portion being anchored to the second valve body.

Valves described herein are favorably configured and arranged such that, in use, when the valve stem is in its non-dispensing position, the pre-metering region is in communication either continuously or transiently with the contents of the aerosol container and the metering chamber is in communication at least transiently with the pre-metering region to allow substance to pass from the aerosol container to the metering chamber, and when the valve stem is in its dispensing position, the pre-metering region is isolated from the metering chamber and a communication path is provided between the metering chamber and the outside of the valve and aerosol container assembly, said path being either continuously or transiently open to allow substance to pass from the metering chamber to the outside.

Once again by reference to the view and embodiment shown in Figure 3, it can be recognized that the exemplary valve includes a second valve body (230), preferably in the form of a cage, defining a pre-metering region (223). The compliant biasing member (215) is in the form of a stack spring. The second valve body (230) and the compliant biasing member (215) are integrally provided in a single integral component (221) where the biasing member is located at least in part in the pre-metering region (223). In the illustrated embodiment, preferably the valve stem (214) is provided as part of the single integral component (221) together with the second valve body (230) and compliant biasing member (215). It will be appreciated that in other embodiments the valve stem may be provided separately; however it is advantageous to provide the valve stem, the second valve body and the compliant biasing member in a single integral component so as to further minimize the overall number of separate components. It is advantageous for the valve stem and compliant biasing member to be thus integrally formed, so that support for the integral component so formed is provided when assembled into a valve, i.e. to prevent lateral relative movement or separation of the valve stem and compliant biasing member.

If the valve stem and compliant biasing member are not to be integrally formed, as is for example the case in the embodiment shown in Figure 9b, embodiments similar to those in the other ensuing figures may be designed with a suitable boundary between these parts, particularly where the compliant biasing member is a compression spring. In such embodiments the bias generally prevents separation of the parts. The boundary may comprise inter-engaging structure such as protrusions and indentations to prevent relative lateral movement. Where the compliant biasing member is a tension spring, inter-engaging features may be provided to allow the valve stem to grip the innermost end of the tension spring to prevent separation of the parts.

Advantageously compliant biasing members are spring members. Depending on the particular valve design, compliant biasing members may be compression or tension spring members. It is well known that compression springs become shortened (e.g. are compressed) when pressure is applied to them, generally offering resistance to a compressive force applied axially, while tension springs become extended (e.g. are stretched) when pressure is applied, generally offering resistance to an extensive force applied axially. Such spring members may have an overall cylindrical, frusto-conical, hourglass (convex), or barrel (concave) shape. They may have a conventional coil or helical, leaf, or stack spring configuration. Commercial pressured metered dose inhalers generally comprise a helical metal coil, cylindrical, compression spring, although other types of spring are known to be applied to aerosol valves in the patent literature, e.g. EP 1477234 and EP 1565270 both mention a stack spring for metering valves. Compliant biasing members may alternatively have less conventional spring configurations, such as C-springs.

Favorably, the single integral component comprising the second valve body, the compliant biasing member, and if applicable the valve stem may comprise a polymeric material, for example with one or more polymeric materials in a composite and/or including other materials such as fillers and/or reinforcing agents in one or more of the polymeric materials. Such components may be favorably molded, in particular via one-shot molding or two or more shot moldings. Alternatively such integral single component may favorably comprise metal and a polymeric material, for example when the compliant biasing member is made of metal (e.g. metal per se) or is reinforced with a metallic sub-structure. Herein the single component may be desirably molded by insert molding in conjunction with one-shot or two or more shot polymeric molding (e.g. insert a metal spring into the appropriate portion of the component mold and then mold, if applicable overmold, the polymeric elements of the component). Examples of suitable polymers for use to make such integral components include acetal (polyoxymethylene) polymers (such as Delrin™ (Dupont de Nemours & Company)), polyetherimide (e.g. ULTEM 1000), liquid crystalline polymer (LCP) or polyetheretherketone (PEEK). Other polymeric materials exhibiting low levels of creep include polyvinylidene difluoride (PVDF), polycarbonate, polyethersulphone (PES) and phenolic laminates. Preferably, food-grade or pharmaceutical-grade materials would be used, although standard industrial grades could be used for non-pharmaceutical applications. Suitable fillers and/or reinforcing agents include fibers, such as glass fibers or carbon fibers. In the event that the compliant biasing member is made of metal per se or is reinforced with a metallic sub-structure, suitable metals include stainless steel.

A cage or cage-like form for second valve bodies is advantageous in that such form is discontinuous and allows for large gaps and ready access of medicament formulation to the internal pre-metering region defined by such bodies as well as facilitating injection molding.

As stated *supra*, valves described herein favorably include an inner seal and an outer seal. Generally the outer seal is desirably located at or near the open end of the first valve body away from the interior, while the inner seal is favorably located at or near the open end of the first valve body towards the interior. The outer seal may be in sliding sealing engagement with the valve stem. Preferably the inner seal is in the form of a lip seal, which allows formation of a relatively low friction sliding seal with the valve stem component during valve operation. Such a seal is partially pressure-assisted during valve firing, in that the pressure difference that develops across it as the metering chamber empties helps to maintain a good seal while the stem (214) remains pushed in towards the aerosol container. Use of a lip seal type of inner seal also facilitates easy pressure-filling of the system. (Pressure filling, or previous air-blowing, can be used to ensure that the inner seal is in its correct as-shown orientation, e.g. if valve assembly turns it 'inside out'.)

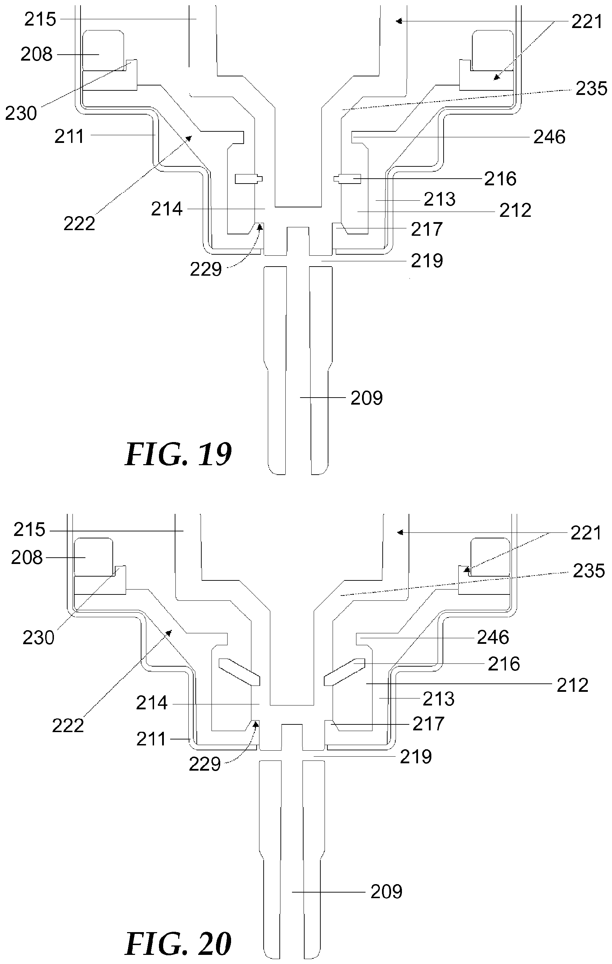

In alternative, "fast-fill, fast-empty" versions of the valves of the present invention, as opposed to "retention valve" versions, the inner seal may be provided as a face seal to operate in a manner analogous to the valves disclosed in WO04/022142 or WO04/022143, as shown later in the examples shown in Figures 19 to 21.

Valve stems typically comprise a dispensing passage, and desirably are movable relative to the inner and outer seals, such that, in use, in the non-dispensing position of the valve stem the dispensing passage is isolated from the metering chamber, and a communication path is provided between the aerosol container and the metering chamber, said path being either continuously or transiently open to allow substance to pass from the aerosol container to the metering chamber, and
in the dispensing position of the valve stem said communication path between the aerosol container and the metering chamber is isolated and the dispensing passage is in communication, either continuously or transiently, with the metering chamber to allow substance to be dispensed from the metering chamber through the dispensing passage.

Again referring to the view and embodiment in Figure 3, it can be seen that the valve stem (214) includes a dispensing passage (209), and at the inner end of the metering chamber there is an inner seal (216) in the form of a lip seal, while at the opposite, outer end of the metering chamber there is an outer seal (217). In this embodiment, both seals are in non-transient, sliding engagement with the valve stem (214). Near the closed end of the dispensing passage is a side hole (219), and in the inner bore of the inner portion of the valve stem is another side hole (228). The two side holes are termed the dispensing side hole and filling side hole, respectively.

Reference is now made to Figures 6 to 8 to explain the positions of the valve stem in operation. It should be noted that these illustrations show the lower half of the valve in Figure 3, but from an angle rotated 90° around the longitudinal axis of the stem, so that the passages of the side holes (219, 228) can be recognized. To ease in viewing and comparison of the positioning, only Figure 7 has been marked with reference numbers. First looking from Figure 6 showing the valve in its rest position to Figure 8 showing the valve at its dispensing (firing) position, it will be noted that the valve stem has moved inwardly towards the aerosol container. Moreover, the exemplary valve is similar to industry-standard push-to-actuate pMDI valves in that, in use, the valve stem is moved axially, e.g. by the user or a breath actuated mechanism, inwardly towards the aerosol container. As mentioned previously, the compliant biasing member (215) is biasing (outwardly away from the aerosol container) the valve stem (214) from its dispensing position towards its rest position. At the rest position of this exemplary valve, the dispensing passage (209) is isolated from the metering chamber since the dispensing side hole (219) is on the outside of the canister at this position. Also there is a communication path between the aerosol container and the metering chamber (212) because there is a communication path from the metering chamber via the filling side hole (228) through the upper stem portion of the valve stem and the pre-metering region (223). When the user starts to actuate the valve by causing the valve stem to push inwardly, the filling side hole (228) and the dispensing side hole (219) start to pass by the inner seal and outer seal (216, 217), respectively. Figure 7 shows a position between the rest position and dispensing position where the metering chamber is completely isolated. As the user continues to actuate and as the side holes completely pass the respective seals, the dispensing passage (209) of the valve stem (214) is in communication with the metering chamber to allow substance to be dispensed from the metering chamber through the dispensing passage (Figure 8). This is a result of the dispensing side hole (219) being now located within the metering chamber (212). Also the metering chamber is isolated from the pre-metering region in the position shown in Figure 8, since the filling side hole (228) is no longer located in the metering chamber. The end point of the inward movement of the valve stem is provided by the design of the compliant biasing member. In particular this is achieved by designing the member such that the different arms of its stack-spring design come to rest solidly against one another, preventing further deformation of the member and thus limiting travel of the valve stem. After firing and once the user lets the valve return to its rest position, the valve stem (214) under the action of the compliant biasing member (215) moves outwardly (away from the interior of the aerosol container) and the filling side hole (228) will enter the now empty metering chamber (212) establishing once again a communication path between the metering chamber and the interior of the aerosol container and thus allowing substance to pass from the aerosol container to the metering chamber.

Valves described herein may advantageously include the first valve body and the inner seal or the outer seal or both seals integrally provided in a single integral component ("second component") to yet further minimize the overall number of components. Alternatively for valves using an inner seal that operates as a face seal, the inner seal may be provided as an integral member of the component comprising the compliant biasing member and second valve body ("first component"), while the first valve body and outer seal may be either provided separately or be provided integrally in a single second component. An example of such a valve will be discussed below.

In the exemplary embodiment shown in Figures 3 to 8, both the inner and outer seals (216,217) are provided together with the first valve body (213) in an integral component (222). This integral component combines at least three separate functions that are conventionally each provided by separate components. The first two of these functions are inner and outer seals, as will be apparent by analogy with the valve of Figures 1 and 2, whilst the third function is the definition of a metering chamber (212).

Integral components comprising the first valve body and the inner seal and/or the outer seal are favorably composed of a polymeric material. Such integral components may be molded, in particular molded via one-shot molding or two or more shot molding. It will be noted that such integral components (e.g. that of the embodiment of Figures 3 to 8) may require 'bumping' off the molding tool (i.e. the molded components may be required to transiently elastically distort as they are ejected from the mold cavity of the injection molding tool), due to the undercuts in the design (e.g. the undercut of the inner seal (216)). This 'bump off process may be air-assisted. Such components may be formed from readily moldable thermoplastic elastomer (TPE) material or thermoplastic vulcanizate (TPV) material. Preferably, food-grade or pharmaceutical grade materials would be used, although standard industrial grades could be used for non-pharmaceutical applications. Examples of commercial TPE materials are copolymers of butylene terephthalate and polyalkylene ether terephthalate, such as HYTREL SC938 TPE or HYTEL SC948 TPE from Dupont de Nemours & Company, the former material having a Shore D hardness of 30, the latter a Shore D hardness of 40. Examples of suitable TPV materials are polyolefinic dynamic vulcanisates of rubber particles in a thermoplastic, such as SANTOPRENE (Exxon Mobil Corporation), e.g. grade 121-62M100, ester-based thermoplastic polyurethanes, such as ESTANE™, polyether polyamide block copolymers, such as PEBAX™, polyolefin elastomers, such as ENGAGE™, polyethylenebutylenes, such as FLEXOMER™ GERS 1085NT, ethylene alpha olefin copolymers, such as EXACT™, or polymers with included Exxelor™ polymer resins. Other materials include co-vulcanisates of ethylenevinylacetate with butyl rubber or a halobutyl rubber and a rubber selected from Neoprene, ethylene propylene diene monomer (EPDM), EPR and polypropyleneoctene, as disclosed in WO2003/18432. Other materials include those disclosed in GB2410500 (seals comprising 1. elastomeric alkene 2. thermoplastic 3. sensitisor eg acrylate), WO2006/092618 (TPE seal material having propylene units with isotactic crystalline form), and GB2323597 (contains TPE, but may not itself be TPE). Alternatively, the components may be composed of rubber materials such as EPDM, Neoprene, Nitrile, Butyl or Chlorobutyl rubber. Surprisingly, such materials can suitably provide the requisite combination of sealing (and low friction sliding seal surfaces) and structural rigidity (and controlled swell in the formulation (4)) for definition of a metering chamber of known volume (and hence of known metered dose size) in a molding of suitable design (such as those of the exemplary designs shown in Figures 3 to 9 and 16 to 21). If desired and/or needed, the polymeric material may be a composite including e.g. fillers and/or reinforcing agents. Advantageously, the polymeric material would have a Shore D hardness of between 25 and 55, more advantageously between 30 and 50 inclusive.

Valves described herein may include a ferrule. Ferrules are typically made by conventional deep-drawing from aluminum alloy strip metal, although alternative forms, such as injection molded plastics, could be used. In a deep-drawn form, the ferrule may comprise a skirt region for attachment onto the neck of the aerosol container by a conventional multi-jaw crimping process. Other processes could however be employed to affix and seal the ferrule to the aerosol container, for example rotary crimping, laser welding or ultrasonic welding. Alternatively, a screw-on attachment could be used, e.g. for an injection molded plastic ferrule.

Valves described herein may further comprise a gasket seal. Such a gasket seal may be provided separately, or once again to aid minimization of the overall number of components it may be provided either as an integral part of the second component, or as an integral part of the first component, or as an integral part of the ferrule.

For valves including a ferrule and a component comprising integrally the first valve body defining at least part of the metering chamber together with the inner seal and/or the outer seal, it is advantageous to size with care said integral component relative to the ferrule due to the intrinsic partially flexible nature of the integral component required for its sealing function. Also, it is advantageous to provide a close radial fit between part of (the inner wall of) the ferrule and part or all of the first valve body's radially outer wall, and to facilitate support of the first valve body in order to help ensure that it cannot flex radially outwardly enough to adversely affect control of the volume of the metering chamber. Accordingly, in such advantageous embodiments, the ferrule is formed such that a portion thereof defines an alcove-space and at least a portion (preferably a substantial portion) of the first valve body is located within the alcove-space such that at least a portion (preferably a substantial portion) of the radially outer wall of the first valve body is adjacent to the inner wall of the portion of the ferrule defining the alcove-space.

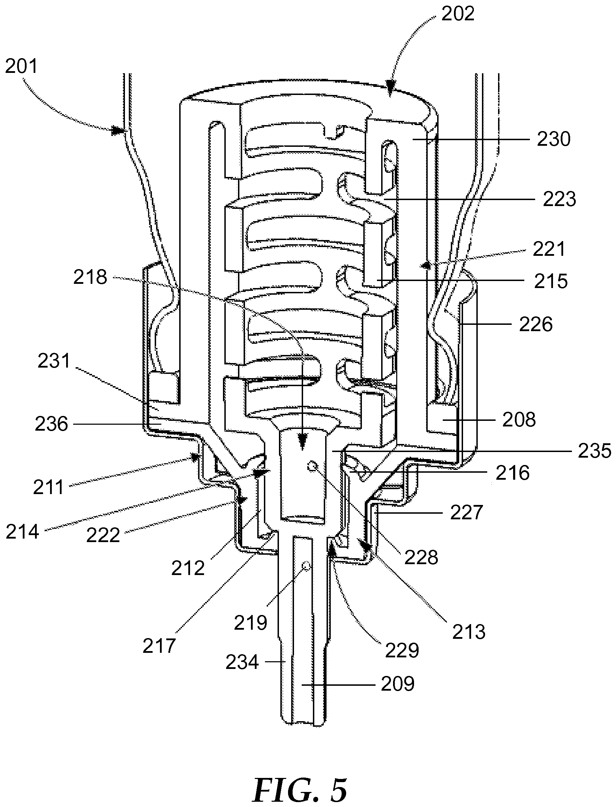

To help ensure a complete understanding of the exemplary embodiment shown in Figure 3 to Figure 8, reference is made to the views shown in Figures 4 and 5. It can be appreciated that the exemplary embodiment of the metered dose dispensing valve includes a total of 4 components, i.e. the first integral component (221), the second integral component (222), the ferrule (211) and the gasket seal (208): half the number of components of the commercial valve shown in Figure 1. For ease in discussion, in the following, the first integral component including the second valve body, the compliant spring member, and, if applicable, the valve stem will be called the antechamber component, and the second integral component including the first valve body together with both the inner and outer seals will be called the metering chamber component.

The antechamber component (221) of the exemplary valve (202) provides multiple integral features and elements. In particular, it provides a valve stem (214) including a hollow male stem part (234) protruding through a piercing in the ferrule (211) and an inner stem part (235) having an internal stem passage (218); a compliant biasing member (215); and a second valve body (230) defining a cage enclosing a pre-metering region (223) and having a gasket rim (231) that gets fixedly located by the valve to vial crimping operation. The cage is discontinuous, with large gaps to allow ready access of medicament formulation to the inside regions (and to facilitate injection molding). For example, the cage (230) can preferably take the form of two curved walls on either side of the compliant biasing member spring (215). The spring (215) is in the form of a plastic stack spring, generally similar in principle to a multilayered metal wave spring.

An internal passage (218) in the inner stem part (235) leads from the interior of the pre-metering region (223) to the metering chamber (212) via the filling side hole (228). The stem dispensing side hole (219) leads from the metering chamber (212) to the dispensing passage (209) provided as a bore of the protruding hollow male stem part (234) when the stem component (214) is pushed in towards the aerosol container (201; see Figure 5 showing a part of the aerosol container). A step on the radially outer surface of the valve stem provides an axial stop (229): this comes into contact with the outer seal (217) when the stem component (214) is pushed outwardly away from the aerosol container (201) by a combination of the compliant biasing member (215) and the medicament formulation vapor pressure, thereby providing both a defined rest position for the valve stem and also an axial seal to minimize formulation leakage during long-term storage. In addition, the valve (202) is preferably designed so that the compliant biasing member (215) provides a measure of outward bias to the valve stem (214), in particular onto the inner stem part (235), when the valve is in its rest position, thus helping to ensure both complete stem return and also good at-rest axial sealing. This outward bias is of course additional to any (temperature-dependent) biasing force provided by the formulation's vapor pressure. It is also worth noting that any temperature dependence of the compliant biasing member (215), for example if it is formed from a polymeric material that appreciably reduces in stiffness as the temperature rises, will tend to be offset by this vapor pressure biasing force tending to increase with rising temperature.

The metering chamber component (222) of the exemplary valve (202) provides multiple integral features and elements, such as a first valve body (213) defining at least in part the metering chamber plus the inner seal (216) and outer seal (217). The component also includes an integral, radially outwardly extending shelf member (236) in the form of a ring (closed or open, i.e. continuous or discontinuous) connected to the radially outer wall of the first valve body. This shelf member may desirably facilitate placement of the antechamber component and/or support of the metering chamber component within the ferrule (in particular near the shoulder of the ferrule) during assembly of the valve. For example, the shelf member can be dimensioned such as to abut the inner walls of the ferrule and thereby to align the antechamber component centrally within the ferrule. Use of a slight dimensional interference fit between the shelf member and the inner walls of the ferrule can serve to align and retain both the antechamber component and the metering chamber component within the ferrule prior to subsequent crimping onto an aerosol container.

The ferrule (211) of the exemplary valve (202) includes a ferrule skirt (226) allowing for crimping onto the canister (201; see Figure 5). A portion of the ferrule (227), in particular the central portion thereof, forms an alcove-space. The ferrule is provided with an opening, typically a piercing, to allow the hollow male stem part (234) to pass through.

To assemble the exemplary valve (202) shown in Figures 3 to 8, the hollow male stem part (234) of the valve stem (214) is pushed through the inner (216) and outer seals (217) of the metering chamber component (222) and the gasket rim (231) of the antechamber component (221) is pushed against the upper face of the radially outer portion of the shelf member (236) of the metering chamber component. Thereafter the hollow male stem part (234) is inserted through the ferrule piercing, with simultaneous placement of a substantial portion of the first valve body (213) into the alcove-space of the ferrule and placement of the lower face of the radially outer region of the shelf member onto the inside of a shoulder of the ferrule. Finally the gasket (208) is placed onto the gasket rim (231) of the antechamber component (221), the valve now being ready for aerosol container (201) crimping and filling. If desired, small nibs in the ferrule's inner walls, and/or a slight crimp, could be used to supplement a dimensional interference-fit as a means of holding the assembled valve together prior to crimping it onto an aerosol container.

Figure 5 also shows an end of the aerosol container and its placement onto the gasket seal (208). (Although not shown, if desired an O-ring like that shown in Figures 1 and 2 (see element with reference number 19) could be placed around the narrow portion of the aerosol container between the aerosol container and the ferrule skirt prior to crimping. Use of such an O-ring could be either in addition to use of a gasket (208) or could be instead of use of such a gasket.) Medicament aerosol formulation would be filled into the aerosol container (201) either before (cold-filling) or after (pressure filling) affixing the valve onto the aerosol container. With only four (or five, when counting an additional O-ring) components to assemble, rather than the eight (or nine, when counting an additional O-ring) of many commercially available valves, the assembly process can be simpler, quicker and cheaper than current pMDI valve assembly processes. In use, referring to Figures 6 to 8 and the description above, the exemplary valve operates in an analogous manner to the prior art conventional retention valve of Figures 1 and 2.

Figures 9a, 10 and 11 illustrate aspects of a second exemplary valve in accordance to the present invention. In this valve, different designs are used for the ante-chamber component (221), the metering chamber component (222) and the ferrule (211), and in addition, no separate gasket seal is used. In regard to the first, as can be appreciated from Figures 10 and 11, which show just the ante-chamber component (221) of this exemplary valve, the compliant biasing member (215) is in the form of an integral coil spring-like member, rather than the stack spring-like member of the exemplary embodiment shown in Figures 3-8. In regard to the other components, referring to Figure 9a the metering chamber component (222) of this exemplary valve (202) includes a first valve body (213) defining at least in part the metering chamber plus the inner seal (216) and outer seal (217). In comparison to the first exemplary embodiment, in this embodiment the inner seal (216) is thicker and more rigid. The component also includes an integral, radially outwardly extending shelf member (236) having a closed ring form connected to the radially outer wall of the first valve body, wherein in this embodiment the shelf-member ends with an U-shaped extension forming an integral gasket seal (238). The ferrule design is altered to take into account differences in the radially outer contours of the metering chamber-component. During assembly, the antechamber component (221) is held onto the shelf member (236) with the integral gasket seal (238) folding over the rim (231) of the antechamber component, and then this sub-assembly is inserted into the ferrule (211), ready for crimping onto an aerosol container (201). Figure 9a shows the valve crimped onto the aerosol container (201). The general principles and operation of this valve are similar to those of the valve shown in Figures 3 to 8. However, in this valve, the end-point of inward movement of the valve stem is provided by the spring-member becoming coil-bound (i.e. by adjacent coils touching). To allow for injection molding of the filling side hole (228) of the stem (214) a molding hole (232; visible in Figures 10 and 11) is provided in the rim (231).

Figure 9b illustrates a different variant of the valve shown in Figures 9a, 10 and 11, being a direct comparison with the illustration of the variant in Figure 9a. In Figure 9b, the valve stem (214) is provided as a separate component to the first integral component (221), said first integral component comprising a compliant biasing member (215) and a second valve body (230) defining a cage enclosing a pre-metering region (223). As in the previous embodiment, the first integral component includes additionally a gasket rim (231) that gets fixedly located by the valve to vial crimping operation. The separate valve stem component (214) has an inner stem part (235) that is retained in a socket at the lower end of the compliant biasing member (215), said biasing member serving to bias the stem (214) towards its non-dispensing position. Whilst the exemplary embodiment of valve (202) shown in Figure 9b thus has one more separate component than does that of the valve of Figure 9a, its design allows the use of differing materials for the stem (214) and for the compliant biasing member (215), for example.

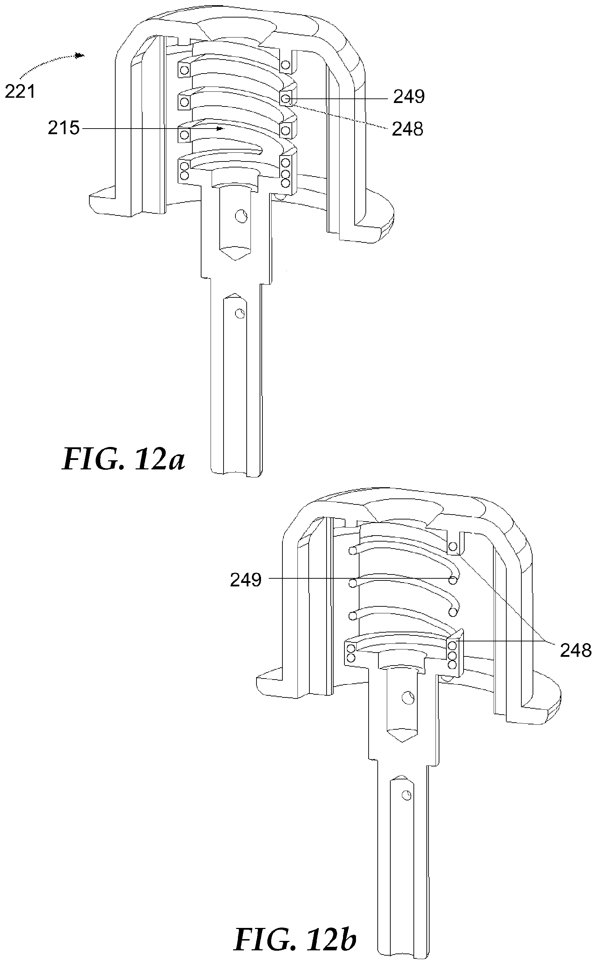

Figures 12 to 15 illustrate alternative ante-chamber components that could be used appropriately in valves similar to the exemplary valve embodiments discussed above.

The ante-chamber component (221) illustrated in Figure 12a is nearly identical to the ante-chamber component shown in Figure 10 except that the component of Figure 12a includes an integral compliant biasing member (215) having a sub-structure (249), in particular a metal spring, within an over-molded (e.g. polymeric) outer structure (248). As mentioned above, such a sub-structure may serve to reinforce the compliant biasing member e.g. facilitating minimization or avoidance of stress relaxation or creep over time. Such integral components may be manufactured by locating a metal element inside the mold for the integral component and then over-molding the chosen polymer material, followed by recovery of the finished component after cooling. Typically the underlying metal element and finished compliant biasing element would have matching structures, e.g. a metal, helical, compression spring substructure for a helical, compression spring member. In a variant of Figure 12a, shown in Figure 12b, the compliant biasing member (215) has just the ends of the sub-structure (249) over-molded with outer structure (248), while the central section is exposed.

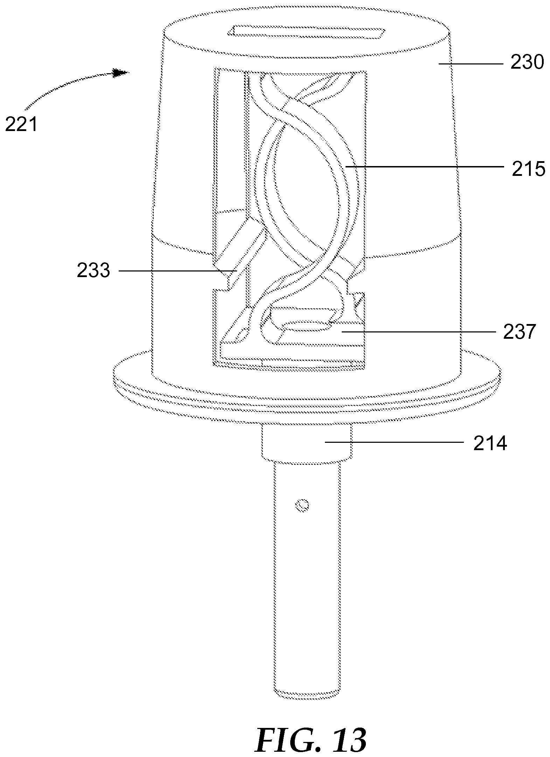

The ante-chamber component (221) illustrated in Figure 13 includes an integral compliant biasing member (215) in the form of two offset C-springs with upper and lower vertical ends. The end point of the inward movement of the valve stem (214) is provided by two horizontal bars (233) located on the interior side of the second valve body (230) and a stop element (237) located between the lower vertical ends of the C-spring-members and the top of the valve stem. When the valve stem is pushed in against the bias, the stop element (237) will travel up and eventually push against the lower surface of the bars (233), thereby limiting the travel of the stem.

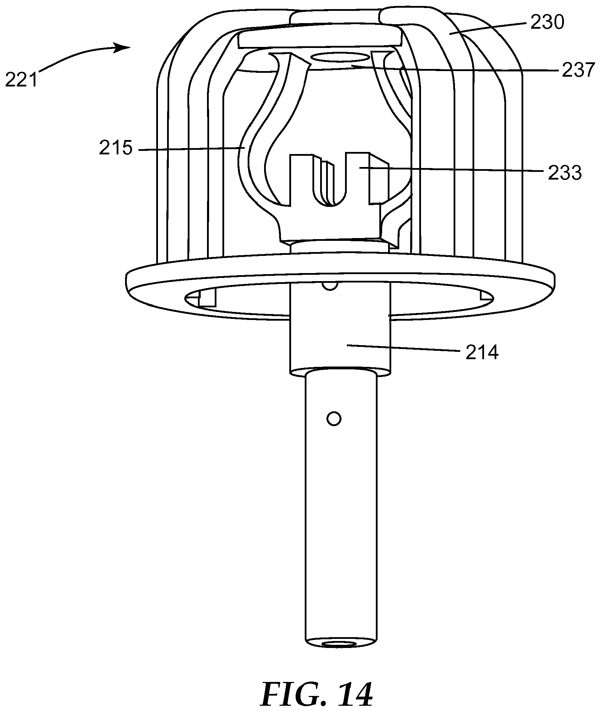

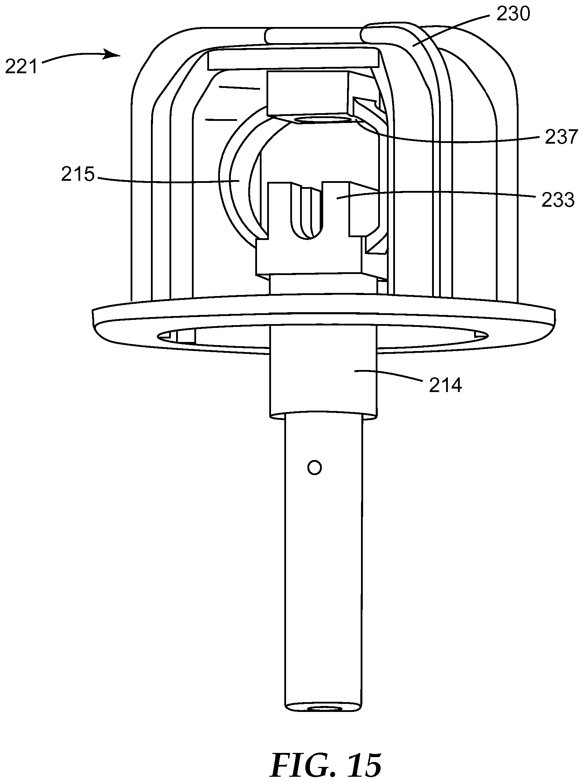

The ante-chamber components (221) illustrated in Figures 14 and 15 include an integral compliant biasing member (215) in the form of two curved and C-shaped arms, respectively. The end point of the inward movement of the valve stem (214) is provided by bars (233) located between the two arms and an opposing stop element (237) located at or near the top of the second valve body (230). When the valve stem is pushed in against the bias, the arms will bow outwardly laterally and the bars (233) will travel up and eventually push against which the lower surface of the stop element (237), thereby limiting the travel of the stem.

Taken together, the Figures and the illustrated exemplary embodiments show that metering valves in accordance to the invention are unique valves suitable for use in pMDIs that show simplicity and low component count (e.g. three or four components in these exemplary valve embodiments, versus typically eight components in many commercially available pMDI valves), whilst providing familiar conventional axial push-to-fire operation and desirably with conventional interfacing to the actuator (via a hollow male valve stem) and to the aerosol container (via a conventional crimp).

As will be apparent to one skilled in the art, it is favorable that compliant biasing members are designed not to impede flow of aerosol formulation as well as to provide appropriate functional parameters, e.g. suitable travels (i.e. distances of stem movement from rest to the firing point, from the firing point to the inwards movement end-point, from the inwards movement end-point back to the valve refill point, etc), adequate reset forces, adequate residual outward biasing force at the rest position, non-excessive firing forces, and a hard-stop feature capable of resisting an appropriate (e.g. 180N) patient force without valve failure and without the valve 'going continuous'. Desirably compliant biasing members are designed such that it is ensured that the aforementioned favorable parameters remain suitable over a desired range of storage and operation temperatures (e.g. 0°C to 45°C), e.g. by finite element analysis and/or by physical testing. For example, Finite Element Analysis (FEA) may be applied to the chosen design of biasing member with a 3-D mesh of about 0.2 mm element size, using well-known principles and methods of optimization described in the book "Finite Element Procedures" by K.J Bathe, published by Prentice Hall in 1996. The dimensions of the design, such as the thicknesses of any horizontal and vertical members and the diameter of any approximately cylindrical profile of the biasing member, may be modified until appropriate force versus distance results are obtained from the FEA. Thus typically where a compression force is applied, at a biasing member compression of 4 mm the force required is about 35 N, and when assembled into a valve the compression is about 1 mm corresponding to a force of about 7 or 8 N. It has been found that readily available FEA software accurately predicts force versus distance profiles for typical biasing members. This information can readily be used to tailor the design of compliant biasing member to provide an acceptable balance of forces, valve stem travel distances, materials stress levels, component costs, and the overall valve size envelope.

To aid in avoiding deposition of medicament and/or to enhance frictional properties for smooth operation, a part or all of the interior surfaces of the metering valve (e.g. part or all of the surfaces of the antechamber component and/or the metering chamber component) may be provided with a low energy surface coating. Example of such coatings include plasma coatings such as DLG (diamond-like glass) as disclosed in WO2009/061895 and WO2010/129753 and perfluoropolyether silane coatings optionally superimposed on a non-metallic, e.g. DLG, base coating as disclosed in WO2009/061891, WO2009/061907, WO2009/061902 and WO2010/129758. Other possible coatings include plasma polymerized fluorinated hydrocarbons, chemical or physical vapour deposited polymers, cold plasma polymerized siloxanes, e.g. dimethylsiloxane, diphenylsiloxane, hexamethyldisiloxane, tetramethyldisiloxane, silazanes, alkoxysilanes, Parylene N, fluoroparylene, Parylene C, Parylene D, fluoroacrylates, coatings of perfluoropolyethersilane, perfluoropolyetherphosphate and/or fluoroalkylsilane, where such coatings are deposited either by dipping, spraying or pouring, and causing or allowing the molecular attachment groups to cure, or by plasma deposition, vacuum deposited silica (about 500 nm thick) on steel component surfaces by a process known as the Silcosteel® process, and fluoroalkyl monolayer coatings as described in WO2007/112312.

As mentioned above, it is desirable to provide valves that can interface in a conventional way to typical aerosol aerosol containers used in pressurized metered dose inhalers. Such aerosol containers are typically made of a metal (e.g. aluminum or aluminum alloy or stainless steel). In such cases, typically it is advantageous to use mechanically crimpable ferrules (e.g. ferrules made of metal, such as aluminum or aluminum alloy). Aerosol containers may be made of other materials, such as glass, plastic or ceramics. Aerosol containers may be coated and/or the interior surfaces of the metering valve may be coated on part or all of their interior walls to reduce drug deposition, e.g. with any of the coatings listed in the previous paragraph. Alternatively, a coating may be selected from mixed fluoropolymer and nonfluoropolymer, where the fluoropolymer is e.g. polytetrafluoroethylene (PTFE), copolymerized ethylene tetrafluorethylene (ETFE), copolymerized perfluoroethylene propylene (FEP), perfluorinated polyalkoxyethylene-co-ethylene (PFA), polyvinylidene difluoride (PVDF), polymerized chlorinated ethylene tetrafluoroethylene (CETFE), and the nonfluoropolymer is e.g. a polymer selected from the following families of polymers: polyethersulphone (PES), polyamideimide (PAI), polyphenylenesulphide (PPS), polyamide, amine-formaldehyde thermosetting resin, benzoguanamine and/or polyethyleneglycol (PEG). Preferred options are PTFE-PES, FEP-PES and PFA-PEG. Aluminium aerosol containers may be anodized, and the anodized surface may help other coatings like PTFE or PFA to adhere more firmly to the container. The aerosol container may be coated with a fluoropolymer by electrostatic dry powder coating. Other coatings may include epoxy-phenolic or epoxyurea-formaldehyde linings (e.g. the epoxy/phenol formaldehyde resins described in WO95/17195).

In the event that a plastic aerosol container (e.g. a blow-molded or injection molded plastic container) is used rather than a conventional metal aerosol container, it may be desirable to use a plastic ferrule (e.g. an injection-molded ferrule) instead of a metal one. Here the plastic ferrule and aerosol container may be designed to allow the ferrule to clip or screw onto the aerosol container, or be equipped with co-operating surfaces for ultrasonic, laser or other thermal welding of the ferrule to the aerosol container. Alternatively, adhesives might be used to affix the valve onto the aerosol container.

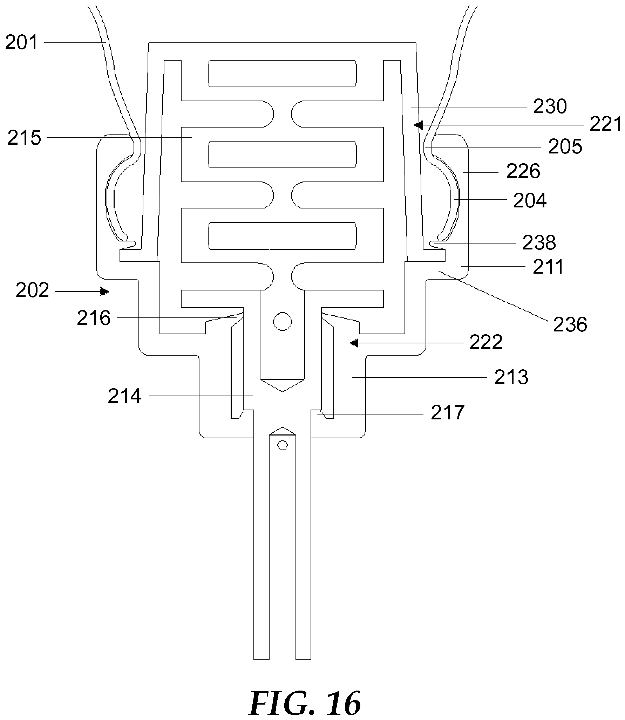

It may be desirable to provide the ferrule as an integral element of the metering chamber component, to advantageously yet further reduce the number of components. The manufacture of such integral components may include single-shot molding or more advantageously at least two-shot molding, e.g. overmolding the other element(s) of the metering chamber component into a formed ferrule or alternatively overmolding a ferrule onto other formed element(s) of the metering chamber component. As can be appreciated from the exemplary embodiment shown in Figure 16, providing the ferrule (and the optional gasket seal) as an integral element(s) of the metering chamber component may allow for the provision of a two-component axial metering valve design.

The exemplary valve (202) illustrated in Figure 16 is shown mounted to an aerosol container (201) and in its rest position. It can be seen that the exemplary embodiment includes an antechamber component (221) including a second valve body (230), compliant biasing member (215) and valve stem (214) of a design similar to that of the antechamber component of the exemplary embodiment shown in Figures 3 to 8. The metering chamber component (222) including a first valve body (213), inner and outer seals (216, 217 respectively) plus a shelf member (236) is also similar to the metering chamber component of the exemplary embodiment shown in Figures 3 to 8, but with a couple of significant differences. In particular, a ferrule (211) together with a gasket seal (238) are formed as integral components of the metering chamber component (222). Desirably the ferrule and integral gasket seal are made of suitably resilient polymeric material (such as SANTOPRENE (Exxon Mobil Corporation), e.g. grade 121-62M100.). The ferrule (211) may be mounted to the container (201) by placement, typically with the container placed on a hard surface with its opening uppermost (i.e. in the opposite orientation to that shown in Figure 16), then applying downward force on the valve. The skirt (226) of the ferrule is thereby displaced radially outwards past a bead (204) near the open end of the container, then upon further downward movement the open end of the skirt moves radially inwardly to grip the neck (205) of the container tightly. The circumferential integral gasket seal (238) forms a pressure tight seal with the open end of the container due to force resulting from the tight grip at the open end of the skirt.

In the previous exemplary embodiments the compliant biasing member is located at least in part (in particular, completely) within the pre-metering region. This is advantageous in that it allows for the production of compact valves with their compliant biasing members protected within their second valve bodies. The latter can be useful in terms of manufacturing, handling and transport of valves and/or individual components thereof. Nonetheless alternative designs are possible where the compliant biasing member may be located in part or completely outside the pre-metering region. This can be appreciated from the exemplary embodiment shown in Figures 17 and 18.

Figures 17 and 18 show isometric views of a sectioned valve (202) attached to a sectioned aerosol container (201), of which part is shown; the valve is shown in its (non-dispensing) rest position (Figure 17) and in its dispensing position (Figure 18). The valve is similar to the valve shown in Figures 9a, 10 and 11 (compare Figures 9a and 17). The ferrule (211) and the metering chamber component (222) including its first valve body (213), inner and outer seals (216, 217), shelf member (236) and integral gasket seal (238) are the same or nearly the same. Also the second valve body (230) and rim (231) of the antechamber component (221) is the same or nearly the same. But the compliant biasing member (215) and the valve stem (214) of the antechamber component show significant differences. In particular, the compliant biasing member (215) is located outside the pre-metering region towards the interior of the container and is configured as a tension, cylindrical helical spring. The valve stem, i.e. the elongated upper portion thereof, passes axially centrally through an interior space defined by the cylindrical spring member, the valve stem and spring member being integrally connected towards the interior end or portion of the spring and valve stem. Due to the extension of the upper portion of the valve, the stem passage (218) with its upper opening (244) extends to the interior of the aerosol container. The upper portion of the valve is provided with a pair of openings (245) to allow for free passage of drug formulation between the pre-metering region and the interior of the upper stem part. As in previous embodiments the upper portion of the valve stem is provided with a filling side hole (228) to allow for filling of the metering chamber and the lower portion of the valve stem with a dispensing side hole (219). Although not visible, an aperture is provided in the second valve body (230) for tooling to form the side filling hole (228) in the upper stem part.

From the user's perspective, the valve operates likes a conventional push-to-fire valve. When the valve is actuated, the valve stem is moved axially and inwardly from its non-dispensing position (Figure 17) to its dispensing position (Figure 18). However, in this valve, the compliant biasing member (215) is a tension spring, which is under tension in the rest position (Fig. 17) and further tensioned in the dispensing position (Fig. 18). In other words, the spring member stretches instead of compressing upon actuation. An extending flange (241) located within the pre-metering region and extending at least part of the way round the elongated upper stem portion (235) of the valve stem (214) prevents the compliant biasing member from over-extending due to excessive inward valve stem movement. When the valve is released, the tension spring moves back to its original size and the valve moves under the action of this bias outwardly from its dispensing position back to its non-dispensing position. When the valve is in its non-dispensing position (Figure 17), the dispensing passage (209) is isolated from the metering chamber (212) and communication paths are provided between the interior of the aerosol container and the pre-metering region (223) and the metering chamber (212) via the filling side hole (228) and its connections to the interior of the aerosol container via the stem passage (218) and top opening (244) and to the pre-metering region via the stem passage (218) and the pair of side openings (245). (It will be recognized that the top opening in the upper stem (218) may optionally be closed, since the metering chamber can be fed solely through the pre-metering region.) In the dispensing position of the valve (Figure 18) the metering chamber (212) is isolated from the pre-metering region (223) and the interior of the aerosol container, since the filling side hole (228) is no longer in communication with the metering chamber, and the metering chamber is in communication with the dispensing passage (209) via the dispensing side hole (219).

Figures 19 to 21 show three exemplary embodiments of valves in accordance with the invention described herein, wherein the inner seal may be provided as a face seal allowing for a "fast-fill, fast-empty" operation. All three Figures show a cross section of just the lower part of the exemplary valve where just a metering chamber component (222) and a gasket seal (208) are shown in their entirety and an antechamber component (221) and ferrule (211) are only partly shown. Please note that, to allow comparison to the fully illustrated embodiment shown in Figures 3 to 8, additional reference numbers are included in Figures 19 to 21.

The exemplary valves shown in Figures 19 and 20 are similar to the exemplary valve shown in Figures 3 to 8; (e.g., compare Figures 19 and 20 to Figure 6), with the exceptions that the upper stem part (235) and the metering chamber component (222) are modified to produce a face sealing valve. The upper stem part (235) of the valve stem (214) is provided with an integral compliant seal, the inner seal (216), extending circumferentially around the upper valve stem part at a position such that in the assembled valve the inner seal is located within the metering chamber. Figures 19 and 20 show two potential different forms and positions of the inner seal. The interior open end of the first valve body (213) is provided as a substantially non-compliant inward circumferential ledge (246). In operation of these valves, as the valve stem is moved axially inwardly from its non-dispensing position to its dispensing position, the inner seal (216) will abut the circumferential ledge (246) of the first valve body (213) thereby making a face seal and sealing off the metering chamber from the pre-metering region. As the valve stem is further moved axially inwardly the seal flexes outwardly, maintaining the face seal, and the dispensing side hole (219) passes the outer seal (217) to move into the metering chamber (212), allowing for dispensing of the contents of the metering chamber via the dispensing passage (209). In the return movement of the valve stem (214) under the influence of the biasing member (215; only partially shown), the dispensing side hole (219) descends into the envelope of the outer seal (217), thereby isolating the metering chamber (212) from the dispensing passage (209). As the valve stem moves further to its rest position, the inner seal (216) disengages from the ledge (246), thereby allowing the metering chamber to refill.

The compliant inner seal (216) is preferably formed by 2-shot moulding into an annular recess provided in the upper stem part (235). When the valve is assembled, the first integral component (221) is inserted by pushing the bottom tip of the stem into the aperture (the insertion aperture) in the radially inward ledge (246), then pushed firmly with the axial stop (229) against the outer seal (217). In the process, the compliant seal (216) deforms and also passes though the insertion aperture, opening out to resume its shape once it has passed through the insertion aperture. The frustoconical profile of the inner seal in the exemplary embodiment shown in Figure 20 is angled away from the direction of insertion upon assembly, thereby facilitating assembly but also providing a more positive inner seal and reducing the possibility for the seal to push back through the insertion aperture, e.g. during pressure filling of drug formulation through the valve stem, during manufacture and filling of the aerosol canister.

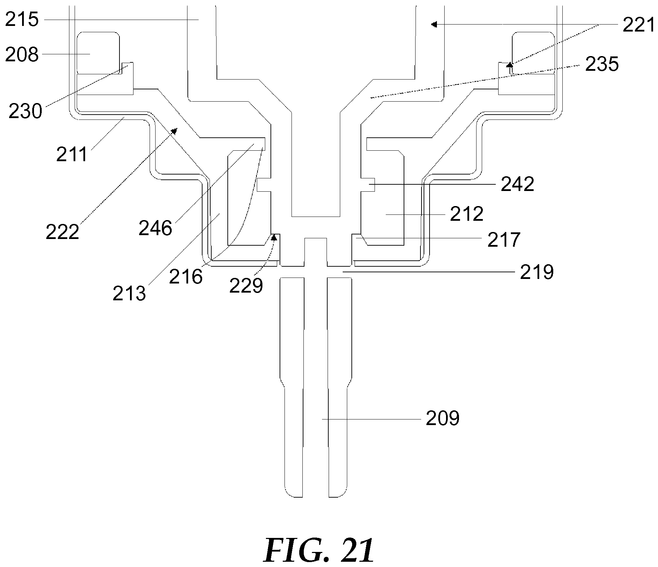

The exemplary valve shown in Figure 21 includes a rigid flange (242) on the upper stem part (235) located within the metering chamber (212) in the assembled valve, and the inner seal (216) is provided as a radially inwardly extending, compliant ledge as an integral element of the metering chamber component (222). Again as the valve is actuated, the valve stem is moved axially inwardly such that the flange (242) on the valve stem will abut the inner seal thereby making a face seal. As the valve is moved further to its dispensing position the inner seal flexes inwardly maintaining the face seal and the dispensing side hole (219) passes the outer seal (217) to move into the metering chamber (212), allowing for dispensing of the contents of the metering chamber via the dispensing passage (209). Assembly is carried out similarly to that for the valves shown in Figures 19 and 20, except that it is the radially inward ledge (246) that deforms such that the opening it defines expands to allow the rigid flange (242) to pass through. Thereafter, the radially inward ledge flips back to the position shown in Figure 21, entrapping the flange.

It will be appreciated that an alternative form of the exemplary valve shown in Figure 21 can provide the compliant, ledge-like inner seal as an integral element of the antechamber component, for example connected to the second valve body via an outwardly radially extending shelf.

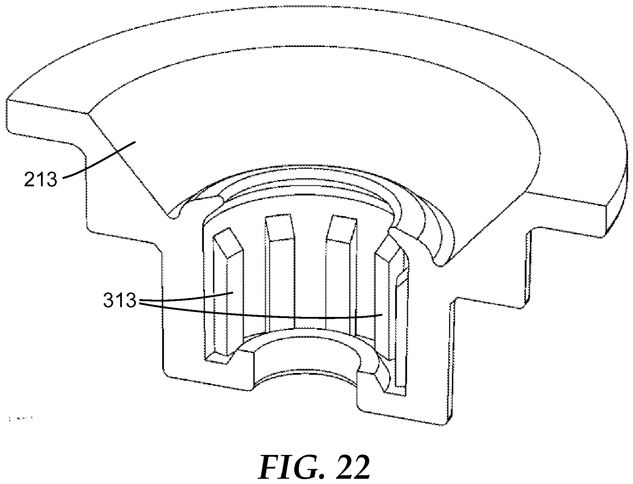

An alternative exemplary embodiment of the first valve body 213 is illustrated in Figure 22. The first valve body 213 has, about the inner circumference in the valve stem receiving part of the first valve body 213, a plurality of ribs 313 that are distributed around the circumference and are intended to guide and support the valve stem 214 when inserted into the first valve body 213. Such an alternative first valve body 213 may be used in the exemplary embodiment shown in Figures 3 to 5 or 9a, for example.

Although not illustrated, it will be appreciated that once a metering valve of one of the types described herein is affixed to an aerosol container, a valve and aerosol container assembly (a "canister") is provided such that the inner walls of the aerosol container and the outer envelope of the metered dose valve located within the aerosol container define a formulation chamber in which medicinal aerosol formulation may be contained.

Canisters fitted with a metering valve described herein may be advantageously utilized as part of dispensers for the administration of medicament through oral, nasal, transmucosal (e.g. buccal, sublingual), vaginal, rectal, ocular or aural delivery. Canisters fitted with a metering valve described herein are particularly suited for delivering medicaments by inhalation to a patient. Accordingly, metering valves described herein and canisters fitted with such valves are particularly suitable for use in or as pressurized metered dose inhalers, respectively.

As indicated above, desirably valves described herein are used in standard pressurized metered dose inhalers, and thus it is favorable that the valves have appropriately dimensioned features to interface with the neck of a standard container (although atypical containers could be provided at the expense of new deep drawing tooling and new feed lines and transfer housings for the container making machine). The neck of a typical container has an opening of about 17 mm diameter comprising a rim and a neck, with a bead between the rim and the neck. Consequently, it would be favorable to provide valves such that the widest part of the valve that would be inserted into the container upon mounting the valve on the container to form a canister (in other words the widest insertable width) would be less than 17 mm in diameter. A gasket is typically included in the valve to seal against the rim, although as illustrated above other means for attachment may be used while maintaining the same outer profile of inhaler unit for use in typical actuators. The depth of the smallest, presently commercially used can is about 28 mm from rim to recessed base. Consequently, it would be favorable to provide valves such that that the longest part of the valve that would be inserted into the container upon mounting the valve on the container to form a canister (in other words the insertable depth of the valve) would be less than 28 mm.

Medicinal aerosol formulations may include any drug or combination of drugs that can be delivered by an aerosol (e.g. administered by inhalation) and such drug or drugs can be provided in suspension and/or solution in liquefied propellant, in particular liquefied HFA 134a and/or HFA 227. If desired or deemed necessary, medicinal aerosol formulations may comprise one or more other non-HFA 134a/HFA 227-propellant components, such as excipients, surfactants and suspending aids.

For manufacture of medicinal aerosol canisters filled with a formulation of drug or drugs in suspension, particulate drug in dry powder form may be and is often supplied in micronized form from the producer of the active ingredient. Micronization can be accomplished, e.g., by using a fluid energy mill driven by compressed air, such as shown in 'Drug Delivery to the Respiratory Tract' ed. D.Ganderton and T.Jones, publ. Ellis Horwood, Chichester (1987) pages 89-90, or by repeated stepwise millings or by use of a closed loop milling system.

The primary particle size of drug (e.g. the size upon completion of micronization) generally has a mass median particle diameter of 5 microns or less, and most suitably said mass median diameter is in the range 0.8 to 3 microns, with at least 90 % by mass of the particles having diameters below 5 microns, which can be determined, for example, by using an Andersen Cascade Impactor.

Depending on the particular valve and/or filling system used, aerosol formulation may be filled into the aerosol container either by cold-filling (in which chilled formulation is filled into the aerosol container and subsequently the valve is fitted onto the aerosol container) or by pressure filling (in which the valve is fitted onto the aerosol container and then formulation is pressure filled through the valve into the aerosol container).

Suitable drugs include those for the treatment of respiratory disorders, e.g., bronchodilators, anti-inflammatories (e.g. corticosteroids), anti-allergics, anti-asthmatics, antihistamines, and anti-cholinergic agents. Other drugs such as anorectics, anti-depressants, antihypertensive agents, anti-neoplastic agents, anti-tussives, anti-anginals, anti-infectives (e.g. antibacterials, antibiotics, anti-virals), anti-migraine drugs, anti-peptics, dopaminergic agents, analgesics, beta-adrenergic blocking agents, cardiovascular drugs, hypoglaecemics, immunomodulators, lung surfactants, prostaglandins, sympathomimetics, tranquilizers, steroids, vitamins, sex hormones, vaccines, therapeutic sense or anti-sense nucleic acids, and other therapeutic proteins and therapeutic peptides may also be employed for delivery by inhalation.

Exemplary drugs which may be employed for delivery by inhalation include but are not limited to: albuterol, terbutaline, pirbuterol, fenoterol, metaproterenol, isoproterenol, isoetharine, bitolterol, epinephrine, tulobuterol, bambuterol, reproterol, adrenaline, ipratropium, oxitropium, tiotropium, darotropium, glycopyrronium, aclidinium, umeclidinium, troventol, beclomethasone, betamethasone, flunisolide, budesonide, mometasone, ciclesonide, rofleponide, aminophylline, dyphylline, theophylline, cromolyn sodium, nedocromil sodium, ketotifen, azelastine, ergotamine, cyclosporine, salmeterol, fluticasone, formoterol, procaterol, indacaterol, olodaterol, carmoterol, milveterol, vilanterol, omalizumab, montelukast, zafirlukast, betamethasone sodium phosphate, dexamethasone, dexamethasone sodium phosphate, dexamethasone acetate, prednisone, methylprednisolone acetate, zileuton, insulin, atropine, prednisolone, benzphetamine, chlorphentermine, amitriptyline, imipramine, clonidine, actinomycin c, bromocriptine, buprenorphine, pentamidine, calcitonin, leuprolide, alpha-1 - antitrypsin, interferons, propranolol, lacicortone, triamcinolone, dinoprost, xylometazoline, diazepam, lorazepam, folic acid, nicotinamide, clenbuterol, ethinyloestradiol, levonorgestrel, and pharmaceutically acceptable salts and esters thereof such as albuterol sulfate, formoterol fumarate, salmeterol xinafoate, vilanterol trifenatate, beclomethasone dipropionate, triamcinolone acetonide, fluticasone propionate, fluticasone furoate, tiotropium bromide, leuprolide acetate and mometasone furoate.

Further drugs that may also be delivered by inhalation include but are not limited to aspirin, acetaminophen, ibuprofen, naproxen sodium, buprenorphine hydrochloride, propoxyphene hydrochloride, propoxyphene napsylate, meperidine hydrochloride, hydromorphone hydrochloride, morphine sulfate, fentanyl citrate, oxycodone hydrochloride, codeine phosphate, dihydrocodeine bitartrate, pentazocine hydrochloride, hydrocodone bitartrate, levorphanol tartrate, diflunisal, diamorphine, trolamine salicylate, methadone hydrochloride, nalbuphine hydrochloride, nalorphine, tetrahydrocannabinol, mefenamic acid, butorphanol tartrate, choline salicylate, butalbital, phenyltoloxamine citrate, diphenhydramine citrate, methotrimeprazine, cinnamedrine hydrochloride, meprobamate, ergotamine tartrate, propanolol hydrochloride, isometheptene mucate, dichloralphenazone, sumatriptan, rizatriptan, zolmitriptan, naratriptan, eletriptan, barbiturates (e.g., pentobarbital, pentobarbital sodium, secobarbital sodium), benzodiazapines (e.g., flurazepam hydrochloride, triazolam, tomazeparm, midazolam hydrochloride, lorazepam, buspirone hydrochloride, prazepam, chlordiazepoxide hydrochloride, oxazepam, clorazepate dipotassium, diazepam, temazepam), lidocaine, prilocaine, xylocaine, beta-adrenergic blockers, calcium channel blockers (e.g., nifedipine, diltiazem hydrochloride, and the like), nitrates (e.g., nitroglycerin, isosorbide dinitrate, pentaerythritol tetranitrate, erythrityl tetranitrate), hydroxyzine pamoate, hydroxyzine hydrochloride, alprazolam, droperidol, halazepam, chlormezanone, haloperidol, loxapine succinate, loxapine hydrochloride, thioridazine, thioridazine hydrochloride, thiothixene, fluphenazine hydrochloride, fluphenazine decanoate, fluphenazine enanthate, trifluoperazine hydrochloride, chlorpromazine hydrochloride, perphenazine, lithium citrate, prochlorperazine, lithium carbonate, bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, lidocaine hydrochloride, phenylbutazone, sulindac, penicillamine, salsalate, piroxicam, azathioprine, indomethacin, meclofenamate sodium, gold sodium thiomalate, ketoprofen, auranofin, aurothioglucose, tolmetin sodium, colchicine, allopurinol, heparin, heparin sodium, warfarin sodium, urokinase, streptokinase, altoplase, aminocaproic acid, pentoxifylline, empirin, ascriptin, valproic acid, divalproate sodium, phenytoin, phenytoin sodium, clonazepam, primidone, phenobarbitol, phenobarbitol sodium, carbamazepine, amobarbital sodium, methsuximide, metharbital, mephobarbital, mephenytoin, phensuximide, paramethadione, ethotoin, phenacemide, secobarbitol sodium, clorazepate dipotassium, trimethadione, ethosuximide, doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluoxetine hydrochloride, doxepin hydrochloride, imipramine hydrochloride, imipramine pamoate, nortriptyline, amitriptyline hydrochloride, isocarboxazid, desipramine hydrochloride, trimipramine maleate, protriptyline hydrochloride, hydroxyzine hydrochloride, diphenhydramine hydrochloride, chlorpheniramine maleate, brompheniramine maleate, clemastine, azelastine, cyproheptadine hydrochloride, terfenadine citrate, clemastine, triprolidine hydrochloride, carbinoxamine maleate, diphenylpyraline hydrochloride, phenindamine tartrate, lamivudine, abacavir, acyclovir, gancyclovir, valganciclovir, cidofovir, foscarnet, azatadine maleate, tripelennamine hydrochloride, dexchlorpheniramine maleate, methdilazine hydrochloride, trimprazine tartrate, trimethaphan camsylate, phenoxybenzamine hydrochloride, pargyline hydrochloride, deserpidine, diazoxide, guanethidine monosulfate, minoxidil, rescinnamine, sodium nitroprusside, rauwolfia serpentina, alseroxylon, phentolamine mesylate, reserpine, calcitonin, parathyroid hormone, acitretin, amikacin sulfate, aztreonam, benzydamine, calcipotriol, chloramphenicol, chloramphenicol palmitate, chloramphenicol sodium succinate, ciprofloxacin hydrochloride, clindamycin hydrochloride, clindamycin palmitate, clindamycin phosphate, efalizumab, metronidazole, metronidazole hydrochloride, gentamicin sulfate, lincomycin hydrochloride, tobramycin sulfate, tacrolimus, vancomycin hydrochloride, polymyxin B sulfate, colistimethate sodium, colistin sulfate, tetracycline, griseofulvin, keloconazole, interferon gamma, zidovudine, amantadine hydrochloride, ribavirin, acyclovir, pentamidine e.g. pentamidine isoethionate, cephalosporins (e.g., cefazolin sodium, cephradine, cefaclor, cephapirin sodium, ceftizoxime sodium, cefoperazone sodium, cefotetan disodium, cefutoxime axotil, cefotaxime sodium, cefadroxil monohydrate, ceftazidime, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, cefuroxime sodium, and the like), penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G potassium, penicillin G procaine, methicillin sodium, nafcillin sodium, and the like), erythromycins (e.g., erythromycin ethylsuccinate, erythromycin, erythromycin estolate, erythromycin lactobionate, erythromycin siearate, erythromycin ethylsuccinate, and the like), tetracyclines (e.g., tetracycline hydrochloride, doxycycline hyclate, minocycline hydrochloride, GM-CSF, ephedrine, pseudoephedrine, ammonium chloride, androgens (e.g., danazol, testosterone cypionate, fluoxymesterone, ethyltostosterone, testosterone enanihate, methyltestosterone, fluoxymesterone, testosterone cypionate), estrogens (e.g., estradiol, estropipate, conjugated estrogens), progestins (e.g., methoxyprogesterone acetate, norethindrone acetate), levothyroxine sodium, human insulin, purified beef insulin, purified pork insulin, glyburide, chlorpropamide, glipizide, tolbutamide, tolazamide, rosiglitazone, pioglitazone, troglitazone, clofibrate, dextrothyroxine sodium, probucol, lovastatin, rosuvastatin, niacin, DNase, alginase, superoxide dismutase, lipase, calcitonion, alpha-1-antitrypsin, interferons, sense or anti-sense nucleic acids encoding any protein suitable for delivery by inhalation, erythropoietin, famotidine, cimetidine, ranitidine hydrochloride, omeprazole, esomeprazole, lanzoprazole, meclizine hydrochloride, nabilone, prochlorperazine, dimenhydrinate, promethazine hydrochloride, thiethylperazine, scopolamine, sildenafil, vardenafil, cilomilast, imiquimod or resiquimod. Where appropriate, these drugs may be delivered in alternative salt forms.

Excipients may include for example, surfactants, co-solvent suspending aids, and/or particulate bulking agents.

Suitable surfactants include those disclosed in EP 372777, GB 837465 and GB 994734. Span 85, oleic acid and/or lecithin are commonly used in medicinal aerosol formulations. Other suitable surfactants for use in medicinal aerosol formulations include HFA-soluble fluorocarbons such as those referred to in WO 91/11173, GB 2263064, as well as polyethyleneoxide, polyoxyethylene-oxypropylene block copolymers such as members of the the Synperonic PE series (Croda International pic), polyoxypropylenes, polyoxyethylene-polyoxypropylene-ethylenediamine copolymers such as members of the Synperonic T series, castor oil ethoxylates such as Alakasurf CO-40, acetylated monoglycerides (e.g. Myvacet 9-40 or 9-45 from Farma International), polyvinyl pyrrolidone, polyvinylacetate, polyvinyl alcohol, polymers of acrylic acid, methacrylic acid and copolymers thereof, polyoxyethylene glyceryl trioleate (TagatTO), polyoxyethylene glyceryl monooleate (TagatO or TagatO2 from Degussa), diol-diacids such as those disclosed in WO 94/21228, oligolactic acid and derivatives thereof, such as those disclosed in WO 94/21229, functionalized PEGs such as those disclosed in WO 2003/059317, amide-ester excipients such as those disclosed in WO 2003/059331, propoxylated PEG (Antarox 31R1 from Solvay), polyoxyethylene glycerol esters such as those disclosed in US 5536444, protective colloids such as those described in WO 95/15151, glyceryl triesters, capr(yl)ic diglyceryl succinates (e.g. Miglyol 829 from Condea Chemie GmbH), Vitamin E acetate, tocopherol (Vitamin E), polyglycolized polyglyceride (e.g. Labrafac Hydro WL 1219 from Gattefosse, Gennevilliers, France), polypropylene glycol, polyethylene glycol e.g. PEG300, aminoacids or derivatives such as disclosed in US 6136294, and other surfactants in the same chemical family as the above but differing in chain length of alkyl or polyalkoxy groups.

Suitable co-solvents may include ethanol, propanol, isopropanol, and other alcohols, glycerol, polyethylene glycol 400, propylene glycol, decanol, sorbitol, mannitol, lactitol, maltitol, glycofurol, dipropylene glycol, propylene glycol diesters of medium chain fatty acids (e.g. Miglyol 840), triglyceride esters of medium chain fatty acids (e.g. Miglyol 810, 812), perfluorocyclobutane, perfluoropentane, perfluorodimethylcyclobutane, menthol, eucapyptus oil, propylene glycol monolaurate (Lauroglycol), diethylene glycol monoethyl ester (Transcutol), isopropyl myristate, saturated hydrocarbons in liquid form and essential oils. Ethanol is commonly used in medicinal aerosol formulations.

Suitable suspending aids may include lactose, glucose, sucrose, D(+)trehalose, as well as their various hydrates, anomers and/or enantiomers, other saccharides such as D-galactose, maltose, D(+)raffinose pentahydrate, sodium saccharin, polysaccharides such as starches, modified celluloses, dextrins, dextrans, DL-alanine, other aminoacids or derivatives such as disclosed in US 6136294, ascorbic acid, sodium sulphate, cetyl pyridinium chloride or bromide other salts e.g. sodium chloride, calcium carbonate, sodium tartrate, calcium lactate, or other organic compounds e.g. urea or propyliodone.

## Claims

1. A metered dose dispensing valve for dispensing metered volumes of an aerosol formulation from an aerosol container, said valve comprising
a first valve body defining in part a metering chamber;
a valve stem passing axially through the metering chamber, movable relative to the chamber between non-dispensing and dispensing positions, and biased from its dispensing position towards its non-dispensing position by a compliant biasing member; and
a second valve body defining at least in part a pre-metering region;
wherein the biasing member has at least one portion engaging the valve stem, thereby imparting a bias thereto, and at least one other portion being anchored to the second valve body; wherein, in use, the valve stem is moved axially against said bias from its non-dispensing position into its dispensing position and upon release the valve stem moves under the action of the bias from its dispensing position back to its non-dispensing position; and **characterised in that** said biasing member and second valve body are integrally provided in a single component.

2. A valve according to claim 1, wherein, the valve is configured and arranged such that, in use, the valve stem is moved axially inwardly towards the aerosol container from its non-dispensing position to its dispensing position and upon release moves outwardly under the action of the bias from its dispensing position to its non-dispensing position.

3. A valve according to any one of the preceding claims, wherein said valve stem is integrally provided together with the biasing member and second valve body in said component.

4. A valve according to any one the preceding claims, wherein the valve further comprises an inner seal and an outer seal, in particular relative to an affixed container, wherein the outer seal is located at or near the open end of the first valve body away from the interior of the container, and wherein the inner seal is located at or near the open end of the first valve body towards the interior of the container.

5. A valve according to claim 4, wherein said valve stem comprises a dispensing passage, the valve stem being movable relative to said seals, such that,
in the non-dispensing position of the valve stem, the dispensing passage is isolated from the metering chamber, and a communication path is provided between the aerosol container and the metering chamber, said path being either continuously or transiently open to allow substance to pass from the aerosol container to the metering chamber, and
in the dispensing position of the valve stem, said communication path between the aerosol container and the metering chamber is closed and the dispensing passage is in communication, either continuously or transiently, with the metering chamber to allow substance to be dispensed from the metering chamber through the dispensing passage.

6. A valve according to claim 4 or 5, wherein said component is a first component and wherein the first valve body and the inner seal, or the first valve body and the outer seal, or the first valve body and both seals, are integrally provided in a single second component.

7. A valve according to claim 4 or 5, wherein said component is a first component, wherein the inner seal is provided as an integral member of the first component and wherein the first valve body and outer seal are either provided separately or are provided integrally in a single second component.

8. A valve according to any one of the preceding claims, wherein the valve further comprises a gasket seal.

9. A valve according to claim 8, wherein the gasket seal is provided as a separate component or as an integral part of the first component.

10. A valve according to claim 8 as dependent on any one of claims 6 or 7 , wherein the gasket seal is provided as an integral part of the second component.

11. A valve according to any one of the preceding claims, wherein the compliant biasing member is located partially within the pre-metering region or completely within the pre-metering region.

12. A canister comprising an aerosol container and a valve according to any one of the preceding claims.

13. A canister according to claim 12, wherein the canister contains an aerosol formulation, in particular a medicinal aerosol formulation, more particularly a pressurized, medicinal aerosol formulation, most particularly a pressurized aerosol formulation comprising drug or a combination of drugs in liquefied propellant, in particular liquefied HFA 134a and/or HFA 227.

14. A medicinal delivery device comprising a valve according to any one of claims 1 to 11 or a canister according to claim 12 or 13.

15. A device according to claim 14, wherein the device is an inhalation device, in particular a pressurized medicinal inhalation device.

## Patentansprüche

1. Abgabeventil für eine abgemessene Dosierung zum Abgeben abgemessener Volumina einer Aerosolformulierung aus einem Aerosolbehälter, wobei das Ventil aufweist:
einen ersten Ventilkörper, der teilweise eine Messkammer definiert;
einen Ventilschaft, der axial durch die Messkammer geht, der relativ zur Kammer zwischen einer Nichtabgabe- und Abgabeposition beweglich ist und durch ein nachgiebiges Vorspannelement von seiner Abgabeposition zu seiner Nichtabgabeposition vorgespannt ist; und
einen zweiten Ventilkörper, der mindestens teilweise einen Vormessbereich definiert;
wobei das Vorspannelement mindestens einen Abschnitt, der mit dem Ventilschaft in Eingriff steht, wodurch ihm eine Vorspannung verliehen wird, und mindestens einen weiteren Abschnitt aufweist, der am zweiten Ventilkörper verankert ist; wobei im Gebrauch der Ventilschaft axial gegen die Vorspannung von seiner Nichtabgabeposition zu seiner Abgabeposition bewegt wird und sich bei Freigabe der Ventilschaft unter der Wirkung der Vorspannung von seiner Abgabeposition zurück zu seiner Nichtabgabeposition bewegt; und **dadurch gekennzeichnet, dass**
das Vorspannelement und der zweite Ventilkörper integral in einer einzigen Komponente vorgesehen sind.

2. Ventil nach Anspruch 1, wobei das Ventil so konfiguriert und angeordnet ist, dass der Ventilschaft im Gebrauch von seiner Nichtabgabeposition zu seiner Abgabeposition axial nach innen zum Aerosolbehälter bewegt wird und sich bei Freigabe unter der Wirkung der Vorspannung von seiner Abgabeposition zu seiner Nichtabgabeposition nach außen bewegt.

3. Ventil nach einem der vorhergehenden Ansprüche, wobei der Ventilschaft zusammen mit dem Vorspannelement und dem zweiten Ventilkörper integral in der Komponente vorgesehen ist.

4. Ventil nach einem der vorhergehenden Ansprüche, wobei das Ventil ferner eine innere Dichtung und eine äußere Dichtung aufweist, insbesondere bezüglich eines befestigten Behälters, wobei die äußere Dichtung an oder nahe dem offenen Ende des ersten Ventilkörpers entfernt vom Inneren des Behälters angeordnet ist, und wobei die innere Dichtung an oder nahe dem offenen Ende des ersten Ventilkörpers zum Inneren des Behälters angeordnet ist.

5. Ventil nach Anspruch 4, wobei der Ventilschaft einen Abgabekanal aufweist, wobei der Ventilschaft bezüglich der Dichtungen beweglich ist, so dass
in der Nichtabgabeposition des Ventilschafts der Abgabekanal von der Messkammer getrennt ist, und ein Verbindungsweg zwischen dem Aerosolbehälter und der Messkammer vorgesehen ist, wobei der Weg entweder dauerhaft oder vorübergehend offen ist, um es zu ermöglichen, dass Substanz vom Aerosolbehälter zur Messkammer geht, und in der Abgabeposition des Ventilschafts der Verbindungsweg zwischen dem Aerosolbehälter und der Messkammer geschlossen ist und der Abgabekanal dauerhaft oder vorübergehend mit der Messkammer in Verbindung steht, um es zu ermöglichen, dass Substanz aus der Messkammer durch den Abgabekanal abgegeben wird.

6. Ventil nach Anspruch 4 oder 5, wobei die Komponente eine erste Komponente ist und wobei der erste Ventilkörper und die innere Dichtung, oder der erste Ventilkörper und die äußere Dichtung, oder der erste Ventilkörper und beide Dichtungen, integral in einer einzigen zweiten Komponente vorgesehen sind.

7. Ventil nach Anspruch 4 oder 5, wobei die Komponente eine erste Komponente ist, wobei die innere Dichtung als ein integrales Element der ersten Komponente vorgesehen ist und wobei der erste Ventilkörper und die äußere Dichtung entweder getrennt vorgesehen sind oder integral in einer einzigen zweiten Komponente vorgesehen sind.

8. Ventil nach einem der vorhergehenden Ansprüche, wobei das Ventil ferner eine Pressdichtung aufweist.

9. Ventil nach Anspruch 8, wobei die Pressdichtung als eine getrennte Komponente oder als ein integraler Teil der ersten Komponente vorgesehen ist.

10. Ventil nach Anspruch 8, wenn von einem der Ansprüche 6 oder 7 abhängig, wobei die Pressdichtung als ein integraler Teil der zweiten Komponente vorgesehen ist.

11. Ventil nach einem der vorhergehenden Ansprüche, wobei das nachgiebige Vorspannelement teilweise innerhalb des Vormessbereichs oder vollständig innerhalb des Vormessbereichs vorgesehen ist.

12. Behälter, der einen Aerosolbehälter und ein Ventil nach einem der vorhergehenden Ansprüche aufweist.

13. Behälter nach Anspruch 12, wobei der Behälter eine Aerosolformulierung, vorzugsweise eine Arznei-Aerosolformulierung, besonders bevorzugt eine unter Druck stehende Arznei-Aerosolformulierung, ganz besonders bevorzugt eine unter Druck stehende Aerosolformulierung enthält, die ein Arzneimittel oder eine Kombination von Arzneimitteln in einem verflüssigten Treibmittel, insbesondere verflüssigtem HFA134a und/oder HFA227 aufweist.

14. Arzneiverabreichungsvorrichtung, die ein Ventil nach einem der Ansprüche 1 bis 11 oder ein Behälter nach Anspruch 12 oder 13 aufweist.

15. Vorrichtung nach Anspruch 14, wobei die Vorrichtung eine Inhalationsvorrichtung, insbesondere eine unter Druck stehende Arznei-Inhalationsvorrichtung ist.

## Revendications

1. Clapet de distribution doseur pour l'administration de volumes dosés d'une formulation d'aérosol contenue dans un récipient d'aérosol, ledit clapet comprenant
un premier corps de clapet définissant en partie une chambre de dosage ;
une tige de clapet traversant axialement la chambre de dosage, déplaçable par rapport à la chambre entre une position de non-administration et une position d'administration, et contrainte de sa position d'administration vers sa position de non-administration par un élément de contrainte élastique ; et
un deuxième corps de clapet définissant au moins en partie une zone de pré-dosage ;
où l'élément de contrainte a au moins une partie de mise en prise de la tige de clapet, sollicitant celle-ci, et au moins une autre partie ancrée dans le deuxième corps de clapet ; où, en cours d'utilisation, la tige de clapet est déplacée axialement contre la contrainte de sa position de non-administration vers sa position d'administration et où un relâchement ramène la tige de clapet de sa position d'administration vers sa position de non-administration sous l'effet de la contrainte ; **caractérisé en ce que** l'élément de contrainte et le deuxième corps de clapet sont réalisés d'un seul tenant comme composant unique.

2. Clapet selon la revendication 1, où ledit clapet est prévu et construit de telle manière qu'en cours d'utilisation, la tige de clapet est déplacée axialement vers l'intérieur dans la direction du récipient d'aérosol, de sa position de non-administration vers sa position d'administration, et est déplacée par relâchement vers l'extérieur, de sa position d'administration vers sa position de non-administration sous l'effet de la contrainte.

3. Clapet selon l'une des revendications précédentes, où la tige de clapet est réalisée d'un seul tenant avec l'élément de contrainte et le deuxième corps de clapet dans le composant.

4. Clapet selon l'une des revendications précédentes, où le clapet comprend en outre un joint intérieur et un joint extérieur, en particulier par rapport à un récipient fixé, le joint extérieur étant situé sur l'extrémité ouverte du premier corps de clapet ou à proximité de celle-ci, à distance de l'intérieur du récipient, et le joint intérieur étant situé sur l'extrémité ouverte du premier corps de clapet ou à proximité de celle-ci, vers l'intérieur du récipient.

5. Clapet selon la revendication 4, où la tige de clapet comprend un passage d'administration, la tige de clapet étant mobile par rapport aux joints, de telle manière
qu'en position de non-administration de la tige de clapet, le passage d'administration est isolé de la chambre de dosage, et un chemin de communication est prévu entre le récipient d'aérosol et la chambre de dosage, ledit chemin étant ouvert soit de manière continue soit de manière temporaire pour permettre le passage d'une substance du récipient d'aérosol à la chambre de dosage, et
qu'en position d'administration de la tige de clapet, le chemin de communication entre le récipient d'aérosol et la chambre de dosage est fermé et le passage d'administration est en communication, continue ou temporaire, avec la chambre de dosage pour permettre à une substance d'être administrée depuis la chambre de dosage par le passage d'administration.

6. Clapet selon la revendication 4 ou la revendication 5, où le composant est un premier composant et où le premier corps de clapet et le joint intérieur, ou le premier corps de clapet et le joint extérieur, ou le premier corps de clapet et les deux joints, sont réalisés d'un seul tenant sous la forme d'un deuxième composant unique.

7. Clapet selon la revendication 4 ou la revendication 5, où le composant est un premier composant, où le joint intérieur est prévu en tant qu'élément intégré du premier composant et où le premier corps de clapet et le joint extérieur sont soit réalisés séparément, soit réalisés d'un seul tenant sous la forme d'un deuxième composant unique.

8. Clapet selon l'une des revendications précédentes, où le clapet comprend en outre un joint d'étanchéité.

9. Clapet selon la revendication 8, où le joint d'étanchéité est prévu comme composant séparé ou comme partie intégrée du premier composant.

10. Clapet selon la revendication 8 si dépendante de la revendication 6 ou de la revendication 7, où le joint d'étanchéité est prévu comme partie intégrée du deuxième composant.

11. Clapet selon l'une des revendications précédentes, où l'élément de contrainte élastique est disposé en partie à l'intérieur de la zone de pré-dosage ou entièrement à l'intérieur de la zone de pré-dosage.

12. Récipient, comprenant un récipient d'aérosol et un clapet selon l'une des revendications précédentes.

13. Récipient selon la revendication 12, où ledit récipient contient une formulation d'aérosol, notamment une formulation d'aérosol à usage médical, en particulier une formulation d'aérosol à usage médical sous pression, tout particulièrement une formulation d'aérosol sous pression comprenant un médicament ou une combinaison de médicaments dans un propulseur liquéfié, en particulier HFA 134a et/ou HFA 227 liquéfiés.

14. Dispositif médical d'administration, comprenant un clapet selon l'une des revendications 1 à 11 ou un récipient selon la revendication 12 ou la revendication 13.

15. Dispositif selon la revendication 14, où ledit dispositif est un dispositif d'inhalation, en particulier un dispositif médical d'inhalation sous pression.
